(19) Europäisches Patentamt / European Patent Office / Office européen des brevets

(11) **EP 4 209 246 B1**

(12) **EUROPEAN PATENT SPECIFICATION**

(45) Date of publication and mention
of the grant of the patent:
**18.02.2026 Bulletin 2026/08**

(21) Application number: **22200390.7**

(22) Date of filing: **07.10.2022**

(51) International Patent Classification (IPC):
***A61M 37/00*** *(2006.01)*

(52) Cooperative Patent Classification (CPC):
**A61M 37/0015;** A61M 2037/0053; Y02A 50/30

(54) **METHOD OF MANUFACTURING OF A PARTICLE-ATTACHED MICRONEEDLE**

VERFAHREN ZUR HERSTELLUNG VON EINER ARTIKELBEFESTIGTEN MIKRONADEL

PROCÉDÉ DE FABRICATION D'UNE MICRO-AIGUILLE FIXÉE À DES PARTICULES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **07.01.2022 KR 20220002903**

(43) Date of publication of application:
**12.07.2023 Bulletin 2023/28**

(73) Proprietor: **QuadMedicine, Inc.
Seongnam-si, Gyeonggi-do 13209 (KR)**

(72) Inventors:
• **PARK, Jung Hwan
13522 Seongnam-si (KR)**
• **CHOI, Jeong Eun
18107 Osan-si (KR)**
• **SHIN, Yu Ra
18107 Osan-si (KR)**

(74) Representative: **V.O.
P.O. Box 87930
2508 DH Den Haag (NL)**

(56) References cited:
WO-A1-2014/151654        WO-A1-2016/097756
WO-A1-2020/232394        KR-A- 20200 043 205
US-A1- 2005 197 308      US-A1- 2014 005 606
US-A1- 2016 015 952

• CHOI JEONG-EUN ET AL: "Preparation of particle-attached microneedles using a dry coating process", JOURNAL OF CONTROLLED RELEASE, vol. 351, 1 November 2022 (2022-11-01), NL, pages 1003 - 1016, XP093279368, ISSN: 0168-3659, DOI: 10.1016/j.jconrel.2022.10.003

EP 4 209 246 B1

## Description

## BACKGROUND OF THE DISCLOSURE

### Field of the Disclosure

The present disclosure relates to a particle-attached microneedle and a method of manufacturing the same, and more particularly, to a microneedle to which solid-phase particles containing an active material are attached, and a method of manufacturing the same.

### Description of the Related Art

As examples of conventional microneedles, there are coated microneedles manufactured by dip-coating and mounting a liquid formulation containing a drug on an insoluble microneedle and followed drying the coating solution, dissolving microneedles manufactured by placing a molding solution containing a drug and an additive in a microneedle mold and then drying the solution, and the like. At this time, in a process of preparing a coating solution or a molding solution, a drug should be transformed into a liquid form, and an appropriate viscosity and composition should be set to load a solution containing a fixed amount of the drug into a microneedle.

Specifically, since both coated microneedles and dissolving microneedles are manufactured by a process based on a liquid formulation, there are several limitations such as 1) preparation of a concentrated drug solution, 2) dissolution of additives and drugs in a co-solvent, 3) denaturation of an active drug in a solution during a manufacturing process, 4) preparation of a solution with a viscosity within a range suitable for a coating or molding process, and 5) drying of a liquid formulation. Since a drug should be prepared at a high concentration to obtain a dry formulation containing the clinical content of the drug required for a microneedle patch, a highly concentrated protein in a solution is likely to be denatured. In addition, when a drug or an additive has low solubility in a co-solvent, it is difficult to find a suitable co-solvent and load the desired amount of the drug into a microneedle.

In the process of drying a coating formulation and a molding solution, the phase separation of the drug and the additive should be also considered. In addition, the phase separation may reduce the mechanical strength of dissolving microneedles. The solution may be denatured by exposure to heat or exposure to air during the microneedle manufacturing process. To minimize the denaturation problem, the manufacturing process should be carried out at low temperatures and the appropriate amount of stabilizer should be added to the solution containing the active drug. In the preparation process of the drug in liquid form, it is difficult to prepare the drug in a liquid formulation form due to denaturation of the active material or low solubility of the active material in co-solvent.

A drying process is another consideration in manufacturing an active drug-loaded microneedle. To dissolve the microneedle, the drying process of a molding solution takes several hours to several days. On the other hand, in the case of coated microneedles, a drying time of several minutes to 1 hour is required, which is most of the time required for the entire manufacturing process. Therefore, if an active drug in a solid state can be loaded without preparing a liquid formulation, various chemical and biological drugs can be delivered to the skin without reducing the activity of a drug required in the liquid process.

An additional element of a microneedle for a successful drug delivery system is the time required to apply a microneedle patch. The time required to apply the microneedle patch increases user convenience and improves drug delivery efficiency.

To overcome the limitations of existing microneedles, the present disclosure has manufactured microneedles to which solid-phase particles are attached. The microneedle to which solid-phase particles are attached can quickly deliver a drug to the skin 1) through the development of a dry process in which it is not required to dissolve an active drug in a solvent, 2) time saving required for the drying process, and 3) the placement of the drug on the outer surface of the microneedle.

[Related Art Document]

[Patent Document]

Korean Patent No. 10-1785766, "MANUFACTURING METHOD FOR COATING MICRONEEDLE AND THE COATING MICRONEEDLE MANUFACTURED BY THE METHOD" Furthermore patent publication US 2014/0005606 A1 discloses an embeddable microneedle patch for transdermal drug delivery and a method of manufacturing the same, whilst patent publication US 2005/0197308 A1 discloses a device for the administration of pharmaceutical agents into the skin of the human body.

# EP 4 209 246 B1

## SUMMARY OF THE DISCLOSURE

Therefore, the present disclosure has been made in view of the above problems, and it is an object of the present disclosure to provide a microneedle with a short microneedle length capable of delivering an active material to the epidermal layer and the dermal layer through the microneedle, unlike conventional injection, and a method for manufacturing the microneedle.

It is another object of the present disclosure to provide a microneedle capable of minimizing the patient's rejection of injection by attached drug particles on the microneedle and increasing the patient's compliance with administration, and a method for manufacturing the microneedle.

It is yet another object of the present disclosure to provide a microneedle to which solid drug particles are attached without changing to a liquid formulation, and a method for manufacturing the microneedles. These objects are achieved by means of a method of manufacturing a particle-attached microneedle according to claims 1, 14 and 16 respectively. Preferred embodiments are defined in the dependent claims 2-13, 15 and 17.

In accordance with an aspect of the invention, the above and other objects are accomplished by the provision of a method of manufacturing a particle-attached microneedles, the method including: first coating a microneedle with an adhesive solution to form an adhesive layer; and dipping the microneedle, on which the adhesive layer has been formed, in a coating well filled with solid-phase drug particles to secondarily attach the solid-phase drug particles on the adhesive layer.

In accordance with an embodiment, the method may further include placing the solid-phase drug particles in the coating well.

In accordance with an embodiment, the method may further include, after secondarily attaching the solid-phase drug particles, reduing voids between the secondarily coated solid-phase drug particles, wherein controlling voids means reducing the distance between drug particles.

In accordance with an embodiment, the microneedle secondarily attached with the solid-phase drug particles may be treated with an air blow to remove unattached particles and to increase the adhesion between particles and adhesive layer.

In accordance with an embodiment, the microneedle secondarily attached with the solid-phase drug particles may be pressed on a porous structure to remove empty space and increase adhesion between the solid-phase drug particles.

In accordance with an embodiment, the microneedle secondarily coated with the solid-phase drug particles may be pressed in a polydimethylsiloxane (PDMS) mold to remove empty space and increase adhesion between the solid-phase drug particles.

In accordance with an embodiment, the adhesive solution may include a complex-type curable material and a hardener, wherein the complex-type curable material is polydimethylsiloxane (PDMS) or a medical epoxy bond, and the hardener is a silicone elastomer curing agent or a hardener.

In accordance with an embodiment, a weight ratio of the complex-type curable material and hardener included in the adhesive solution may be 40:1 to 20:1. An adhesive strength of 0.05 N/cm or more is recommended for adhesive layer.

In accordance with an embodiment, the adhesive solution may include a single-type curable material, and the single-type curable material may be any one selected from the group consisting of polyethyleneglycol (PEG), polyethylene oxide (PEO), beeswax, triglyceride, silicone, epoxy, protein bioadhesive and a UV-curable medical-grade epoxy resin.

In accordance with an embodiment, the adhesive solution may include an adhesive material and a solvent, wherein the adhesive material is any one selected from the group consisting of sugar 2-20%, glucose 25-50%, starch 1-4%, gelatin 1-4%, carboxymethylcellulose (CMC-Na) 1-4%, gum arabic 2-5%, cellulose derivative (hydroxypropyl cellulose (HPC), hydroxypropylmethylcellulose (HPMC)) 1-4%, methyl cellulose (MC), ethyl cellulose 0.5-2%), polyvinylpyrrolidone (PVP) 2-5%, cyanoacrylate, a fibrin glue, a protein glue, a polyethylene glycol (PEG) hydrogel sealant, silicone, epoxy, protein bioadhesive and a mussel adhesive protein, and the solvent is water or an organic solvent.

In accordance with an embodiment, a raw material of the microneedle may be one or more biodegradable polymers selected from poly(lactic acid), poly(L-lactic acid), poly(glycolic acid), poly(lactic-co-glycolic acid), and non-biodegradable/biocompatible polymer(non-biodegradable but biocompatible polymer) of a cyclic olefin copolymer, polycarbonate, nylon, polyethylene, and polypropylene.

In accordance with an embodiment, the microneedle may have a length of 50 $\mu$m to 1,000 $\mu$m.

In accordance with an embodiment, an active material of the solid-phase drug particles may include any one selected from the group consisting of interferon, erythropoietin (EPO), follicle-stimulating hormone (FSH), parathyroid hormone (PTH), granulocyte-macrophage colony-stimulating factor (G-CSF), granulocyte-macrophage colony-stimulating factor (FM-CSF), human chorionic gonadotropin, progesterone, calcitonin, glucagon, GNRH antagonist, insulin, human growth hormone (GHD), testosterone, lidocaine, diclofenac, oxybutynin, ketoprofen, alendronate, enapril maleate, phenylpropanolamine, cromolyn, isotretinoin, oxytocin, paroxetine, flurbiprofen, sertraline, venlafaxine, leuprolide, risperidone, galantamine, enoxaparin, etanercept, fentanyl, filgrastim, heparin, somatropin, and sumatriptan, or may include any one selected from the group consisting of a vaccine containing any one of Hepatitis A, B, and C, HIV, influenza, diphtheria, tetanus, pertussis, Lyme disease, rabies, pneumococcus, yellow fever, cholera, vaccinia, tuberculosis, rubella, measles, mumps, rotavirus, botulism, and herpes virus, and a botox toxin and their mixture.

Active ingredient particles are prepared by milling, spary drying or milling of lyophilized product.

In accordance with another aspect of the invention, there is provided a method of manufacturing a particle-attached microneedle, the method including: first coating a microneedle with an adhesive solution to form an adhesive layer; and dispersing the solid-phase drug particles on the adhesive layer using a micro-sieve to secondarily attach the solid-phase drug particles on the adhesive layer.

In accordance with an embodiment, the method may further include, after secondarily coating the solid-phase drug particles, reducing voids(empty space) between the secondarily coated drug particles.

In accordance with an embodiment, to reduce the voids between the drug particles, the microneedle secondarily attached with the drug particles may be treated with an air compressor or mechanical vibrator.

In accordance with an embodiment, to reduce the voids between the drug particles, the microneedle secondarily attached with the drug particles may be dipped on a puff with drug particles.

In accordance with an embodiment, to reduce the voids between the drug particles, the microneedle secondarily attached with the drug particles may be pressed in a polydimethylsiloxane (PDMS) mold.

In accordance with an embodiment, the adhesive solution may include a complex-type curable material and a hardener.

In accordance with an embodiment, the adhesive solution may include a single-type curable material.

In accordance with an embodiment, the adhesive solution may include an adhesive material and a solvent.

In accordance with another aspect of the invention, there is provided a method of manufacturing a particle-attached microneedle, the method including: first coating a microneedle with an adhesive solution to form an adhesive layer; and dipping the microneedle, on which the adhesive layer has been formed, in a petri dish filled with solid-phase drug particles to secondarily attach the solid-phase drug particles on the adhesive layer.

In accordance with an embodiment, the method may further include, after secondarily coating the solid-phase drug particles, reducing voids(empty space) between the secondarily attached solid-phase drug particles.

In accordance with an embodiment, to control the voids between the drug particles, the microneedle secondarily attached with the drug particles may be treated with an air compressor.

In accordance with an embodiment, to control the voids between the drug particles, the microneedle secondarily attached with the drug particles may be dipped on a puff with drug particles.

In accordance with an embodiment, to control the voids between the drug particles, the microneedle secondarily attached with the drug particles may be pressed in a polydimethylsiloxane (PDMS) mold.

In accordance with an embodiment, the adhesive solution may include a complex-type curable material and a hardener.

In accordance with an embodiment, the adhesive solution may include a single-type curable material.

In accordance with an embodiment, the adhesive solution may include an adhesive material and a solvent.

## BRIEF DESCRIPTION OF THE DRAWINGS

The above and other objects, features and other advantages of the present disclosure will be more clearly understood from the following detailed description taken in conjunction with the accompanying drawings, in which:

FIG. 1A is a schematic diagram illustrating a particle-attached a microneedle manufacturing method;
FIG. 1B illustrates various particle attachment method;
FIG. 2 illustrates an SEM image (x900) showing the particles used;
FIG. 3A is a perspective view showing components for particle attachment method;
FIG. 3B illustrates a schematic diagram of a particle attachment method;
FIG. 3C is a perspective view illustrating components of a particle attachment method using a micro-sieve;
FIG. 4A illustrates a stereoscopic microscope image of a microneedle having an adhesive layer formed thereon;
FIG. 4B illustrates a stereoscopic microscope image in which particles are attached only to the tip ends of the microneedle after the formation of the adhesive layer of the microneedle;
FIG. 4C illustrates a stereoscopic microscope image of particles attached to the entire microneedle after the formation of the adhesive layer of the microneedle;
FIG. 5A illustrates an SEM image of a uncoated PLA microneedle (x43, side view).
FIG. 5B illustrates an SEM image of a uncoated PLA microneedle (x45, lateral view).
FIG. 5C illustrates an SEM image of a uncoated PLA microneedle (x40, top view).
FIG. 5D illustrates an SEM image of a microneedle on which an adhesive layer has been formed (x45, side view).
FIG. 5E illustrates an SEM image of a microneedle on which an adhesive layer has been formed (x50, lateral view).

FIG. 5F illustrates an SEM image of a microneedle on which an adhesive layer has been formed (x40, top view).

FIG. 5G illustrates an SEM image of a microneedle with particles that are attached only to the tip ends of the microneedle (x45, side view).

FIG. 5H illustrates an SEM image of a microneedle with particles that are attached only to the tip ends of the microneedle (x50, lateral view).

FIG. 5I illustrates an SEM image of a microneedle with particles that are attached only to the tip ends of the microneedle (x40, top view).

FIG. 5J illustrates an SEM image of a microneedle with particles that are attached only to the tip ends of the microneedle (x45, side view).

FIG. 5K illustrates an SEM image of a microneedle with particles that are attached only to the tip ends of the microneedle (x50, lateral view).

FIG. 5L illustrates an SEM image of a microneedle with particles that are attached only to the tip ends of the microneedle (x40, top view).

FIG. 6A illustrates a stereomicroscopic image of the surface of pig skin into which a microneedle with particles attached only to tip ends of the microneedle was administered;

FIG. 6B illustrates a stereomicroscopic image of the surface of pig skin into which a microneedle with particles attached to the entirety of the microneedle was administered;

FIG. 7A illustrates an SEM image (x200) of a microneedle before administration to pig skin, wherein particles are attached only to the tip ends of the microneedle (x200);

FIG. 7B illustrates an SEM image (x200) of a microneedle after administration to pig skin, wherein particles are attached only to the tip ends of the microneedle (x200). It can be confirmed that an adhesive layer of the marked portion does not peel off and maintains an intact shape thereof;

FIG. 8A illustrates an image taken by SEM of a pig skin surface into which a microneedle coated only with an adhesive layer was administered (x35).

FIG. 8B illustrates an image taken by SEM of a pig skin surface into which a microneedle with particles attached only to the tip ends of the microneedle was administered (x35).

FIG. 9 is a graph illustrating the peel force between PDMS 40 and pig skin;

FIG. 10A is a graph illustrating the horizontal work between PDMS 40 and pig skin;

FIG. 10B is a graph illustrating the peel force between PDMS 40, glucose particles, and pig skin;

FIGS. 11A to 11C illustrate images in which ovalbumin particles of different sizes are attached to a PDMS 40 adhesive layer;

FIGS. 12A to 12D illustrate images of observing the Z-stack fluorescence pattern of pig skin over time using a confocal microscope;

FIG. 13 is a graph illustrating a change in fluorescence intensity on the surface of a tip of a microneedle while the microneedle to which fluorescent ovalbumin particles are attached stays on the skin;

FIG. 14A illustrates an optical microscope image of a microneedle to which green glucose particles are attached. FIG. 14B illustrates a fluorescence microscope image of a microneedle to which fluorescent ovalbumin particles are attached;

FIG. 15A illustrates an optical microscope image of a microneedle to which ovalbumin particles are attached, and FIG. 15B illustrates an optical microscope image of a microneedle, to which ovalbumin particles are attached, recovered after administering the microneedle to mouse skin in vivo for 5 minutes;

FIG. 16 illustrates a schematic diagram of an immunization schedule; and

FIG. 17 illustrates a graph of antibody titer when ovalbumin is administered.

## DETAILED DESCRIPTION OF THE DISCLOSURE

The present disclosure will now be described more fully with reference to the accompanying drawings and contents disclosed in the drawings. However, the present disclosure should not be construed as limited to the exemplary embodiments described herein.

The terms used in the present specification are used to explain a specific exemplary embodiment and not to limit the present inventive concept. Thus, the expression of singularity in the present specification includes the expression of plurality unless clearly specified otherwise in context. It will be further understood that the terms "comprise" and/or "comprising", when used in this specification, specify the presence of stated components, steps, operations, and/or elements, but do not preclude the presence or addition of one or more other components, steps, operations, and/or elements thereof.

It should not be understood that arbitrary aspects or designs disclosed in "embodiments", "examples", "aspects", etc. used in the specification are more satisfactory or advantageous than other aspects or designs.

In addition, the expression "or" means "inclusive or" rather than "exclusive or". That is, unless otherwise mentioned or clearly inferred from context, the expression "x uses a or b" means any one of natural inclusive permutations.

In addition, as used in the description of the disclosure and the appended claims, the singular forms "a", "an" and "the" are intended to include the plural forms as well, unless context clearly indicates otherwise.

In addition, when an element of construcions is referred to as being "on a surface of" or "on" another element, the element can be directly on another element or an intervening element can be present.

According to the present disclosure, a drug is intended to be mounted on a microneedle in the form of a solid phase rather than a liquid phase. Most drugs are prepared in the form of particles and are manufactured in a solid phase through various processes. Therefore, when the existing "coating solution or molding method" is used, a drug should be prepared in a solution status. In the solution, an excipient such as a thickener, stabilizer and microneedle matric material should be added, so that a final solidified formulation can be different from approved pharmaceuticals. In addition, it is difficult to prepare a drug in a liquid phase because of undesirable activity loss or the low solubility of an active material when drug particles has low solubility in solution. To solve the problems, the present disclosure intends to deliver a drug or a drug-containing formulation into the skin by loading them on a microneedle as solid particles without dissolving in solvent.

In addition, an conventional method of loading a solid-phase drug onto a microneedle was accomplished by dispersing or enclosing drug particles inside the microneedle matrix or solidified formulation. In this case, particles should be suspended in coating solution or molding solution, and the dissolution characteristics of solid additive in solidified formulation can cause undesirable dissolution property of solidified formulation wih drug particles.

Therefore, to overcome the limitations, the present disclosure intends to present a microneedle manufacturing method adopting "a particle-attached method" so as to deliver an active material into the skin without affecting the dissolution properties of formulation. The particle attachment method refers to attaching drug-containing particles to the outside surface of microneedles with physical adhesion force and may be called "a dry powder coating process" as another term. The advantages and characteristics of "a particle attachment method" compared to an existing "solution coating method" are as follows.

The particle attachment method attaches solid-phase particles directly on the surface of a microneedle without a separate coating solution preparation process, so the coating process is very simple compared to a solution coating method. In this case, there is no need to change a dosage form because drug particles use a powder such as a freezing agent or a solidifying agent, and thus, access to the approval of the Ministry of Food and Drug Safety is easy. Since no additional coating excipient is added to the drug, there is no fear of changing a release rate according to the dosage form change. Since the process of dissolving a drug is omitted, particles can be coated on a microneedle regardless of whether a drug is hydrophilic or hydrophobic, and the powder remaining after coating can be recycled, thereby reducing drug waste. In addition, since a drug before coating is in a solid state, stability is maintained even when exposed to the air for a long time, and since the drug particles are microparticled in the original formulation form thereof and physically attached to the external surface of the microneedle, the drug can be immediately exposed and eluted to body fluid when the microneedle patch is attached to the skin. In addition, since a thickener is not added to the drug, the drug can be absorbed more quickly into the skin after administration through the microneedle.

Hereinafter, an embodiment of the present disclosure will be described in detail with reference to the accompanying drawings.

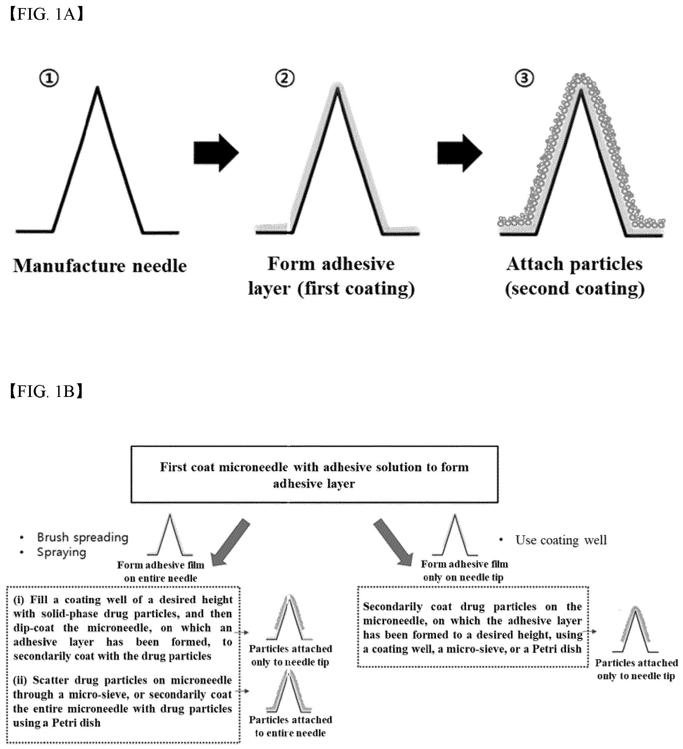

FIG. 1A is a flowchart illustrating a method of manufacturing a particle-attached microneedle according to an embodiment of the present disclosure.

Referring to FIG. 1A, the method of manufacturing a particle-attached microneedle according to an embodiment of the present disclosure is peformed through two steps after manufacturing a microneedle. Here, the steps include a first step of first coating a microneedle with an adhesive solution to form an adhesive layer; and a second step of secondarily coating(attaching) solid-phase drug particles on the adhesive layer to be attached thereto.

FIG. 1B illustrates a method of first coating a microneedle with an adhesive solution to form an adhesive layer; and a method of attaching(coating) drug particles to the microneedle-adhesive layer.

Referring to FIG. 1B, the method of forming the adhesive layer of the particle-attached microneedle according to an embodiment of the present disclosure may be one selected from dip-coating(dipping), brush spreading, and spray coating. However, due to the cumbersomeness and reproducibility of experiments, a brush spreading method or a spray coating method is mainly used when an adhesive layer is formed over the entire needle, and a dip-coating method of mainly using a coating well is recommended when an adhesive layer is formed only at a needle tip.

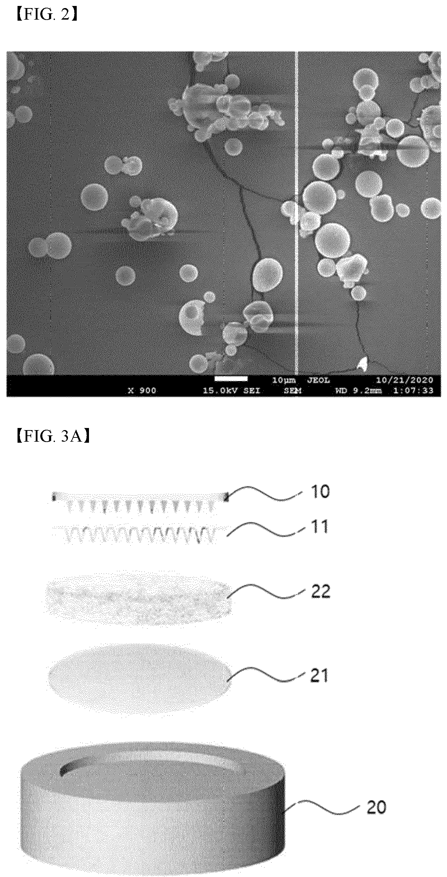

In the present disclosure, a coating well means a cylindrical container, and a single large flat circular groove is dug in a part of an upper part of a cylindrical container. Dug particles may be attached on a needle tip to have a desired height by filling the groove with the drug particles or an adhesive solution, or a needle tip may be supported in an adhesive solution to have a desired height. Referring to FIG. 3A, the shape of a coating well 20 can be confirmed.

The method of first coating a microneedle to form an adhesive layer and the method of attaching drug particles to the microneedle-adhesive layer are descirbed in detail as follows.

First, among methods of first coating a microneedle to form an adhesive layer, a brush spreading method is advantageous in that it is possible to thinly coat a microneedle by coating a solution on the microneedle with a brush dipped in an adhesive solution, but is disadvantageous in that it is difficult to control a height at which an adhesive layer is coated. Spray coating is a method of coating by spraying an adhesive solution with a sprayer and is advantageous in that it is possible to thinly coat, but like the brush spreading method, it is disadvantageous in that it is cumbersome to adjust the coating height of an adhesive layer. A dip-coating method is advantageous in that the height of an adhesive layer can be adjusted, but is disadvantageous in that it is difficult to thinly coat an adhesive layer on a needle because it depends on the viscosity of a solution.

As such, after first coating the microneedle with an adhesive solution to form an adhesive layer, drug particles are secondarily coated on the adhesive layer. When an adhesive layer is first formed over the entire needle using the brush spreading method or the spray coating method, (i) after filling a coating well of a desired height with solid-phase drug particles, the microneedle on which the adhesive layer has been formed is dip-coated and is secondarily coated with drug particles, thereby coating only the needle tip with the drug particles, or (ii) the entire microneedle is secondarily coated with drug particles by scattering (dispersing) the drug particles on a microneedle through a micro-sieve, or (iii) a microneedle is dip-coated with drug particles on a Petri dish to secondarily coat the entire needle with the drug particles.

Meanwhile, since when only the needle tip is first coated to form an adhesive layer by dip-coating with a coating well, an adhesive layer is formed on only the needle tip, any one of a method of dippng drug particles using a coating well, a method of dispersing drug particles using a micro-sieve, and a method of dip-coating with drug particles using a Petri dish may be used to secondarily coat only the needle tip with drug particles.

In the method of manufacturing the particle-attached microneedle, three methods of secondarily coating solid-phase drug particles on the microneedle are described below.

According to the present disclosure, the method of manufacturing the particle-attached microneedle includes a step of first coating a microneedle with an adhesive solution to form an adhesive layer; and a step of dip-coating the microneedle, on which the adhesive layer has been formed, in a coating well filled with solid-phase drug particles to secondarily coat the adhesive layer with the solid-phase drug particles.

Before proceeding the step of first coating to form an adhesive layer, a microneedle is made of a biodegradable polymer or biocompatible polymer (non-biodegradable but biocompatible polymer) and then an adhesive solution is prepared. A particle-attached microneedle includes a patch and a microneedle on the patch. The patch may be formed in a circular shape, and a plurality of pointed pyramid-shaped microneedle tips may be provided on the patch. The microneedle may be inserted through the skin of a person, and after being administered to the skin, the solid particles attached on the microneedle is detached or dissolved for delivery into the skin.

A biodegradable polymer, which is a raw material of the microneedle, may be one or more selected from poly(lactic acid), poly(L-lactic acid), poly(glycolic acid) and poly(lactic-co-glycolic acid). In addition, the microneedle has a length of 50 $\mu$m to 1,000 $\mu$m, preferably a length of 400 $\mu$m to 1,000 $\mu$m. The length of the microneedle represents an appropriate length for the needle to reach the dermis layer through the epidermal layer of the skin.

Non-biodegradable/biocompatible polymer(Non-biodegradable but biocompatible polymer), which is a raw material of the microneedle, may be one or more selected from cyclic olefin copolymer, polycarbonate, nylon, polyethylene, and polypropylene. In addition, the microneedle has a length of 50 $\mu$m to 1,000 $\mu$m, preferably a length of 400 $\mu$m to 1,000 $\mu$m. The length of the microneedle represents an appropriate length for the needle to reach the dermis layer through the epidermal layer of the skin.

Next, the method of forming (first coating) an adhesive layer on the uncoated microneedles may use the following three types.

First, a complex-type curable material and a hardener (the complex-type curable material is a curable resin),
Second, a single-type curable material (the single-type curable material is a liquid curable resin),
Third, an adhesive material with solvent (the adhesive material is a polymer).

For first and second case of curable adhesive material, the curing process for adhesive layer may be completed before drug particles are attached to an adhesive layer, or the curing process for adhesive layer may be completed after attaching the drug particles to an adhesive layer.

For third case of adhesive material with solvent, solvent is removed by drying and then particles are attached on the adhesive layer after removing solvent.

First, when the complex-type curable material and the hardener are used,
the complex-type curable material and the hardener may be a combination of polydimethylsiloxane (PDMS) and a silicone elastomer curing agent (hardener), a combination of a medical epoxy bond and a hardener, or the like.

Second, when the single-type curable material is used,
the single-type curable material does not have a separate a hardener, so after coating the single-type curable material on the microneedle, drug particles are attached thereto, and curing is controlled by temperature (heat) or UV. Examples of a single-type material whose curing is controlled by temperature include polyethyleneglycol (PEG), polyethylene oxide (PEO), beeswax, triglyceride, silicone, epoxy, protein bioadhesive and the like, and examples of a single-type material whose curing is controlled by UV include a UV-curable medical-grade epoxy resin, and the like.

When using the curable material as in the first and second methods, an additional solvent is not required, so that both hydrophilic and hydropobic drug particles can be attached on the adhesive layer.

Third, when a mixture of an adhesive material and a solvent is used, the adhesive material may be sugar 2-20%, glucose 25-50%, starch 1-4%, gelatin 1-4%, carboxymethylcellulose (CMC-Na) 1-4%, gum arabic 2-5%, or the like when the solvent is water, and since the solvent is water, it is suitable for attaching a hydrophobic drug. When the solvent is an organic solvent such as ethanol, the adhesive material may be a cellulose derivative (hydroxypropyl cellulose (HPC), hydroxypropylmethylcellulose (HPMC)) 1-4%, methyl cellulose (MC), ethyl cellulose 0.5-2%), polyvinylpyrrolidone (PVP) 2-5%, cyanoacrylate, a fibrin glue, a protein glue, a polyethylene glycol (PEG) hydrogel sealant, silicone, epoxy, protein bioadhesive, a mussel adhesive protein, or the like. Similarly, adhesion is exhibited within the above % ranges, and since the solvent is an organic solvent, it is suitable for attaching a hydrophilic drug.

In the first case in which a complex-type curable material and a hardener are used as an adhesive solution, the complex-type curable material and the hardener are mixed and prepared in a weight ratio of 40:1 to 20:1, preferably a weight ratio of 40:1 to 30:1. An adhesive strength of 0.05 N/cm or more is recommended for adhesive layer. When the complex-type curable material and the hardener are diluted in a lower ratio than 20:1, the adhesion force of the adhesive solution is significantly reduced, causing ths loss of adhesive force. After attaching drug particles to the microneedle using the adhesive solution, a curing process is performed to attach solid-phase drug particles on the microneedle. An adhesive strength of 0.05 N/cm or more is recommended for adhesive layer.

When the complex-type curable material and the hardener are used together or the single-type curable material is used, a microneedle is dip-coated, brush-spread, or spray-coated with the complex-type curable material and the hardener or the single-type curable material, thereby forming an adhesive layer.

Since the adhesive solution before curing has sticky viscosity, The the dipping of uncoated microneedles in the curing solution for few seconds is recommended . After forming the adhesive layer, curing solution is cured in an oven at 70°C for 1 hour or at room temperature for one day to obtain an adhesive layer on an array bof microneedles. Alternatively, the adhesive layer may be solidified by irradiating ultraviolet (UV) light after using a UV curable resin as the single-type curable material.

After forming the adhesive layer on the particle-attached microneedle, the microneedle is dipped in a coating well filled with solid-phase drug particles to secondarily attach solid-phase drug particles on the adhesive layer. To proceed with this step, it is necessary to prepare drug particles and fill the drug particles into the coating well.

To proceed with the step, a solid-phase drug is dissolved in distilled water or buffer, and then lyophilized. The lyophilized drug is pulverized with a mortar and passed through a micro sieve to prepare drug particles having a uniform shape and size. The drug particles passing through the micro sieve have a diameter of 20 $\mu$m or less.

Preferably, the drug particles have a diameter of 10 nm to 50 $\mu$m. More preferably, the drug particles may have a diameter of 1 $\mu$m to 20 $\mu$m, but the present disclosure is not limited thereto. The efficiency of delivery of particles through the microneedle can be affected by the size of the particles. If the particle size is too large, the amount of delivered particles may be decreased because the drug is pushed out due to increased friction between the particles and the skin during insertion into the skin. Conversely, if particles are too small, the amount of delivered particles may be decreased because the total volume of particles attached to the adhesive layer decreases.

FIG. 2 illustrates an SEM image of drug particles magnified by 900 times. In the image, it can be confirmed that the diameter is 20 $\mu$m or less.

An active material of the drug particles may be any material that can be medicamentally/medically used in the human body, such as a solidifying compound, a lyophilized antibody, a lyophilized drug substance such as a protein, and a vaccine. Specifically, the active material may be any one selected from the group consisting of interferon, erythropoietin (EPO), follicle-stimulating hormone (FSH), parathyroid hormone (PTH), granulocyte-macrophage colony-stimulating factor (G-CSF), granulocyte-macrophage colony-stimulating factor (FM-CSF), human chorionic gonadotropin, progesterone, calcitonin, glucagon, GNRH antagonist, insulin, human growth hormone (GHD), testosterone, lidocaine, diclofenac, oxybutynin, ketoprofen, alendronate, enapril maleate, phenylpropanolamine, cromolyn, isotretinoin, oxytocin, paroxetine, flurbiprofen, sertraline, venlafaxine, leuprolide, risperidone, galantamine, enoxaparin, etanercept, fentanyl, filgrastim, heparin, somatropin, and sumatriptan, or may be any one selected from the group consisting of a vaccine containing any one of Hepatitis A, B, and C, HIV, influenza, diphtheria, tetanus, pertussis, Lyme disease, rabies, pneumococcus, yellow fever, cholera, vaccinia, tuberculosis, rubella, measles, mumps, rotavirus, botulism, and herpes virus, and a botox toxin.

The step of filling the prepared uniformly sized drug particles into the coating well may be any one selected from a method of evenly filling the drug particles by spreading the drug particles using a micro-sieve; a method of filling a coating well with the drug particles and then applying pressure thereto in a vertical direction such that the particles are densely filled; and a method of dispersing the drug particles in an organic solvent that has good volatility and does not dissolve the drug particles, filling a coating well with the dispersed drug particles, and evaporating the organic solvent to leave only the particles.

When the particles are not filled evenly in the step of filling the coating (dipping) well with the solid-phase drug particles, a step of hardening a complex-type curable adhesive material and a hardener to manufacture a film, and then disposing the film on a coating well may be further included before the step of filling the coating well. The complex-type curable adhesive material and hardener constituting the film may be made of any one selected from the same group as that of the complex-type curable material and hardener constituting the adhesive solution for forming the adhesive layer on the surface of the microneedle. That is, the film needs to satisfy the condition that the solid-phase drug particles on the film are evenly dispersed and, to transfer the solid-phase drug particles into the skin successfully without peeling off. Thus, the high adhesion force of the film on polymer microneedles is recommended.

When the complex-type curable material is mixed in a higher ratio than 10:1, there is a problem that the drug particles are attached to the film with low adhesive force. When the weight ratio of matrix material to curing agent increased, the adhesion force of particles to the adhesive layer increases.

The drug particles may be more evenly distributed and attached strongly by filling the particles and proceeding with dipping after placing the film in the coating well. This is because in the case of a hydrophobic drug, static electricity is generated when the particle size is small and drug particles tend to agglomerate when the drug particles are filled in a coating well. As described above, when drug particles are applied to a coating (dipping) well on which a film is spread, static electricity is generated less, so that the drug particles can be evenly dispersed and coated.

Like other curing poly-dimethyl siloxane, the adhesive layer is manufactured by curing it in an oven at 70°C for 1 hour or at room temperature for one day. The cured adhesive film has adhesion force to particles. Therefore, the solid-phase drug particles located on the adhesive layer on the microneedle.

Next, a step of secondarily coating the microneedle, on which the adhesive layer has been formed, with solid-phase drug particles is performed. This step is performed by dipping the microneedle patch, on which the adhesive layer has been formed, into the coating (dipping) well flatly filled with the lyophilized/milled, spray drieddrug particles or the milled drug particles and the film such that the adhesive layerd microneedle tips are evenly attached with the drug particles.

After the step of secondarily attaching the solid-phase drug particles, a step of controlling void (space) between drug particles may be further included (reducing voids). In the present disclosure, controlling the voids between drug particles attached to microneedles means reducing the distance between drug particles.

The method of controlling the voids (space) between the drug particles after attaching drug particles on the microneedles may be any one selected from among a method of treating the microneedle with an air compressor; a method of dip-coating the microneedle onto a puff; and a method of pressing the microneedle in a polydimethylsiloxane (PDMS) mold.

Specifically, the method of treating a microneedle with an air blow is a method of injecting air into a particle-attached microneedle. After this process, unattached drug particles to the adhesive layer are removed by air blow, and additional force for increase of adhesion between particles and adhesive layer was generated by air blow.

In the method of dipipng microneedles onto a puff filled with particles, a puff is a porous structure. When microneedles with adhesive layer was applied on the puff, particles in puff can be transferred on the adhesive layer uniformly.

In the method of pressing particle attaceh microneedles to elastic polydimethylsiloxane (PDMS) mold, the PDMS mold refers to an engraved PDMS mold used to manufacture uncoated microneedle replicate. Since the size and shape of the particle attached microneedles and the PDMS mold perfectly fit each other, the drug particles on the adhesive surface of particle-attached microneedles are compacted and the voids between the particles are reduced by pressing of elastic PDMS rubber.

Through the step of controlling the voids between the drug particles, the solid-phase drug particles may be distributed more thinly and evenly on the surface of the microneedle. Through this manufacturing method, a particle-attached microneedle including the adhesive layer formed by first coating the microneedle with the adhesive solution and secondarily attaching the solid-phase drug particles on the adhesive layer is manufactured. Similarly, the particle-attached microneedle is in a state in which voids between the secondarily coated solid-phase drug particles are controlled. By controlling the voids, a large amount of drug particles may be attached to the same area, and since the drug particles are attached tightly to the surface of the microneedle, the risk of the particles being pushed out when applied to the skin may be reduced.

Since the particle-attached microneedle according to the present disclosure is formed by attaching drug particles on the microneedle, a desired dissolution rate may be determined by controlling the particle size. On the other hand, in an existing microneedle coating method, additional additives such as a stabilizer, a thickener, and an excipient are added to solid-phase drug particles to prepare a liquid formulation for coating, and it is necessary to change the formulation to make a solution. However, the addition of dissolution of drug in co-solvent is not required if the particles themselves without solvation are loaded on the microneedle according to the present disclosure, the loading on the microneedles can be performed more simply, and target drug can be delivered into the skin. In addition, there is no need for a dissolution process of solid-phase drug in a buffer to make a liquid formulation for coating, thus the attachment of particles can be performed regardless of the hydrophilicity or lipophilicity of the drug.

FIG. 3A is a perspective view illustrating components of a particle-attached microneedle manufactured using a dip-coating method according to an embodiment of the present disclosure. Referring to FIG. 3A, the microneedle loaded with solid-phase drug particles according to an embodiment of the present disclosure includes a microneedle 10, an adhesive layer 11, solid-phase drug particles 22, a film 21, and a coating well 20.

In the step of first coating a microneedle with an adhesive solution to form an adhesive layer, the microneedle 10 is dip-coated, brush-spread, or spray-coated with the adhesive solution and cured at 70°C for 1 hour or at room temperature for one day. The cured adhesive layer 11 is first coated on the microneedle 10, and the adhesive layer 11 has adhesion force to the solid-phase drug particles 22. In the step of dip-coating the microneedle, on which the adhesive layer has been formed, in a coating well filled with solid-phase drug particles to secondarily coat the drug particles on the adhesive layer, first, the film 21 is placed on the coating well 20. Due to the film 21, the solid-phase drug particles 22 evenly disposed in the coating well 20. Next, in the process of coating the drug particles, the solid-phase drug particles 22 are secondarily attached on the microneedle 10 while maintaining the solid state thereof without changing the formulation thereof when the microneedle on which the adhesive layer has been formed is dip-coated in the coating well 20 to which the drug particles 22 have been applied.

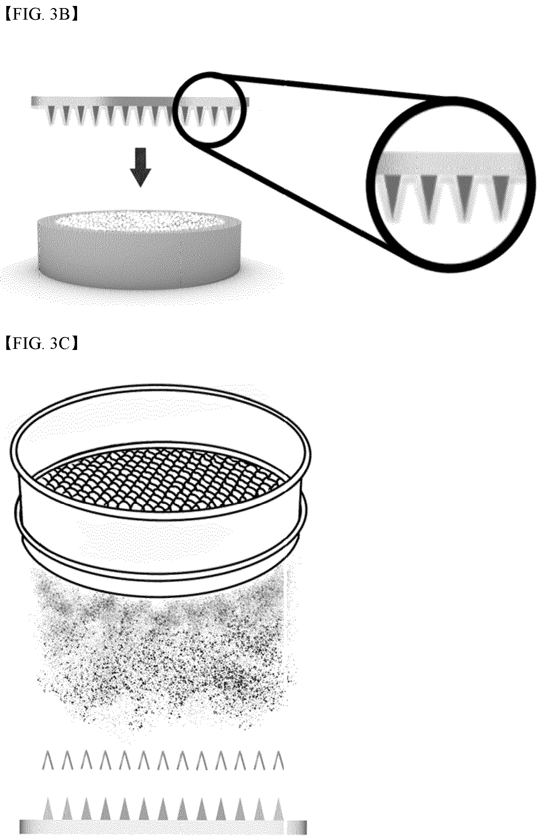

FIG. 3B is a schematic diagram illustrating a method of attaching drug particles and illustrates a combined form of the microneedle 10 and the adhesive layer 11 FIG. 3A and a combined form of the drug particles 22, the film 21, and the coating well 20.

FIG. 3C is a perspective view illustrating a method of attaching drug particles using a micro-sieve.

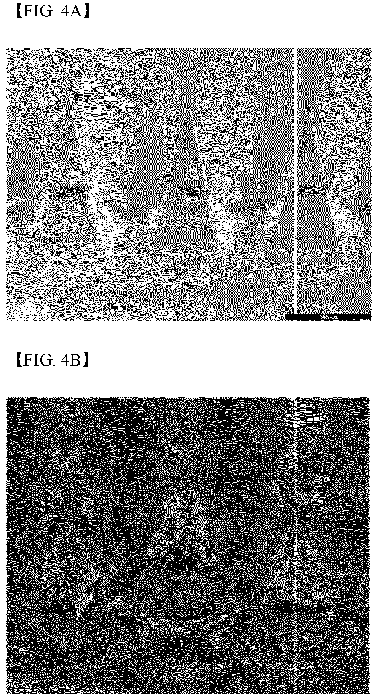

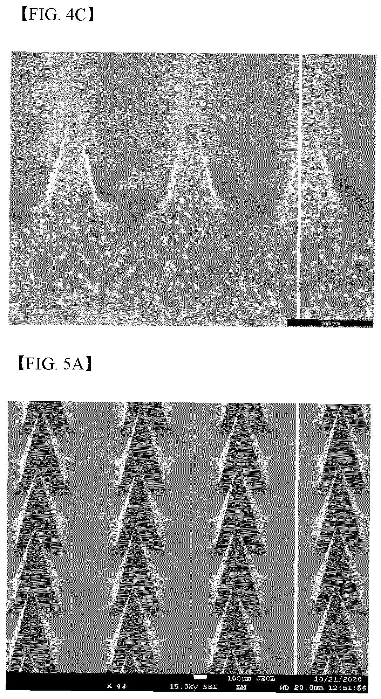

FIG. 4A illustrates a optical microscopic image of microneedles on which the adhesive layer has been formed. Here, the scale bar in the lower right represents 500 μm.

FIG. 4B is a optical microscopic image illustrating the shape of a particle attached microneedles by a microneedle manufacturing method according to an embodiment of the present disclosure are formed only on microneedle tip ends of the microneedle. Here, the magnification is 120 times. Referring to FIG. 3B, it can be confirmed that, by dip-coating the microneedle in the solid-phase drug particles 22 located on a coating well, the solid-phase drug particles are attached on the ends of needle tips of the microneedle as it is as a solid crystal. When a dip-coating method is used for attachment of particles, there is an advantage that the height of particle attachement can be determined.

In the particle-attached microneedle formed as described above, the solid-phase drug particles are not internalized in microneedle tips, but are exposed on the surface of the microneedle tips, thus the drug can be delivered rapidly to the epidermis or dermis by exposure to interstitial fluid in skin. That is, the additives of thickner or microneedle matrix are not added in particle formulation, so the dissolution and elution patterns of the drug particles are not affected by additive.

According to the present disclosure, the method of manufacturing the particle-attached microneedles include a step of first coating a microneedle with an adhesive solution to form an adhesive layer; and a step of dispersing the solid-phase drug particles on the adhesive layer using a micro-sieve to secondarily coat the adhesive layer with the solid-phase drug particles.

Before proceeding the step of first coating to form an adhesive layer, microneedles are made of a biodegradable or non-biodegradable/biocompatible polymer(non-biodegradable but biocompatible polymer) and then an adhesive solution is prepared. A particle-attached microneedle includes a patch and a microneedle array on the patch. The patch may be formed in a circular shape, and an array of plurality of pointed pyramid-shaped microneedle tips may be provided on the patch. The microneedle may be inserted through the skin of a person, and after administration to the skin, the solid paricles on the microneedle is dissolved and delivered into the skin.

A biodegradable polymer, which is a raw material of the microneedle, may be one or more selected from poly(lactic acid), poly(L-lactic acid), poly(glycolic acid), poly(lactic-co-glycolic acid), and non-biodegradable/biocompatible polymer(non-biodegradable but biocompatible polymer) from a cyclic olefin copolymer, polycarbonate, nylon, polyethylene, and polypropylene. In addition, the microneedle has a length of 50 μm to 1,000 μm, preferably a length of 400 μm to 1,000 μm. The length of the microneedle represents an appropriate length for the needle to reach the dermis layer through the epidermal layer of the skin.

Next, the method of forming (first coating) an adhesive layer on the uncoated microneedles may use the following three types.

First, a complex-type curable material and a hardener (the complex-type curable material is a curable resin),
Second, a single-type curable material (the single-type curable material is a liquid curable resin),
Third, an adhesive material and a solvent (the adhesive material is a polymer).

When drug particles are attached to an adhesive layer, the adhesive layer can have remaining solvent for solvent based adhesive or adhesive layer is completely solidified when a curable material is used.

First, when the complex-type curable material and the hardener are used,
the complex-type curable material and the hardener may be a combination of polydimethylsiloxane (PDMS) and a silicone elastomer curing agent (hardener), a combination of a medical epoxy bond and a hardener, or the like.

Second, when the single-type curable material is used,

the single-type curable material does not have a separate a hardener, so after coating the single-type curable material on the microneedle, drug particles are attached thereto, and curing is controlled by temperature (heat) or UV. Examples of a single-type material whose curing is controlled by temperature include polyethyleneglycol (PEG), polyethylene oxide (PEO), beeswax, triglyceride, silicone, epoxy, protein bioadhesive and the like, and examples of a single-type material whose curing is controlled by UV include a UV-curable medical-grade epoxy resin, and the like.

When using the curable material as in the first and second methods, an additional solvent is not required, so that both hydrophilic and hydropobic drug particles can be attached on the adhesive layer.

Third, when a mixture of an adhesive material and a solvent is used, the adhesive material may be sugar 2-20%, glucose 25-50%, starch 1-4%, gelatin 1-4%, carboxymethylcellulose (CMC-Na) 1-4%, gum arabic 2-5%, or the like when the solvent is water, and since the solvent is water, it is suitable for attaching a hydrophobic drug. When the solvent is an organic solvent such as ethanol, the adhesive material may be a cellulose derivative (hydroxypropyl cellulose (HPC), hydroxypropylmethylcellulose (HPMC)) 1-4%, methyl cellulose (MC), ethyl cellulose 0.5-2%), polyvinylpyrrolidone (PVP) 2-5%, cyanoacrylate, a fibrin glue, a protein glue, a polyethylene glycol (PEG) hydrogel sealant, silicone, epoxy, protein bioadhesive, a mussel adhesive protein, or the like. Similarly, adhesion is exhibited within the above % ranges, and since the solvent is an organic solvent, it is suitable for attaching a hydrophilic drug.

When the complex-type curable material and the hardener are used together or the single-type curable material is used, a microneedle is dip-coated, brush-spread, or spray-coated with the complex-type curable material and the hardener or the single-type curable material, thereby forming an adhesive layer.

Since the adhesive solution before curing has sticky viscosity, the the dipping of uncoated microneedles in the curing solution for few seconds is recommended. After forming the adhesive layer, curing solution is cured in an oven at 70°C for 1 hour or at room temperature for one day to obtain an adhesive layer on an array of microneedles. Alternatively, the adhesive layer may be solidified by irradiating ultraviolet (UV) light after using a UV curable resin as the single-type curable material.

To prepare solid-phase drug particles for secondary attachment of particles on adhesive layer, a solid-phase drug is dissolved in distilled water or buffer, and then lyophilized. The lyophilized drug is pulverized with a mortar and passed through a micro sieve to prepare drug particles having a uniform shape and size. The drug particles passing through the micro sieve have a diameter of 20 $\mu$m or less.

Preferably, the drug particles have a diameter of 10 nm to 50 $\mu$m. More preferably, the drug particles may have a diameter of 1 $\mu$m to 20 $\mu$m, but the present disclosure is not limited thereto. The efficiency of delivery of particles through the microneedle can be affected by the size of the particles. If the particle size is too large, the amount of delivered particles may be decreased because the drug is pushed out due to increased friction between the particles and the skin during insertion into the skin. Conversely, if particles are too small, the amount of delivered particles may be decreased because the total volume of particles attached to the adhesive layer decreases.

An active material of the drug particles may be any material that can be medicamentally/medically used in the human body, such as a solidifying compound, an antibody, a drug substance such as a protein, and a vaccine. Specifically, the active material may be any one selected from the group consisting of interferon, erythropoietin (EPO), follicle-stimulating hormone (FSH), parathyroid hormone (PTH), granulocyte-macrophage colony-stimulating factor (G-CSF), granulocyte-macrophage colony-stimulating factor (FM-CSF), human chorionic gonadotropin, progesterone, calcitonin, glucagon, GNRH antagonist, insulin, human growth hormone (GHD), testosterone, lidocaine, diclofenac, oxybutynin, ketoprofen, alendronate, enapril maleate, phenylpropanolamine, cromolyn, isotretinoin, oxytocin, paroxetine, flurbiprofen, sertraline, venlafaxine, leuprolide, risperidone, galantamine, enoxaparin, etanercept, fentanyl, filgrastim, heparin, somatropin, and sumatriptan, or may be any one selected from the group consisting of a vaccine containing any one of Hepatitis A, B, and C, HIV, influenza, diphtheria, tetanus, pertussis, Lyme disease, rabies, pneumococcus, yellow fever, cholera, vaccinia, tuberculosis, rubella, measles, mumps, rotavirus, botulism, and herpes virus, and a botox toxin and their combination.

After the step of secondarily attaching the solid-phase drug particles, a step of controlling void (space) between drug particles may be further included (reducing voids). In the present disclosure, controlling the voids between drug particles attached to microneedles means reducing the distance between drug particles.

The method of controlling the voids between the drug particles after attaching the drug particles on adhesive layer may be any one selected from among a method of treating the microneedle with an air compressor; a method of dip-coating the microneedle onto a puff; and a method of pressing the microneedle in a polydimethylsiloxane (PDMS) mold.

Specifically, the method of treating a microneedle with an air blow is a method of injecting air into a particle-attached microneedle. After this process, unattached drug particles to the adhesive layer are removed by air blow, and additional force for increase of adhesion between particles and adhesive layer was generated by air blow.

In the method of dip-coating a microneedle onto a puff, a puff is a porous structure with voids for storage of particles. When particle-attached microneedles are pressed with a puff, empty space between the drug particles are compacted by the puff so that the particles can be more densed.

In the method of pressing a particle attached microneedles to a polydimethylsiloxane (PDMS) mold with same geometric cavity, the PDMS mold refers to an engraved PDMS mold used to manufacture a microneedle 10. Since the size and shape of the microneedle 10 and the PDMS mold perfectly fit each other, the drug particles are compacted and the voids between the particles of the particle-attached microneedles are reduced without use of air blow.

Through the step of controlling the voids between the drug particles, the solid-phase drug particles may be attached more evenly on the surface of the adhesive layer of microneedle. Through this manufacturing method, the adhesive layered microneedles are formed by first coating the uncoated microneedle with the adhesive solution and then the solid-phase drug particles are attached on the adhesive layer. Similarly, the particle-attached microneedles have empty space between the initial attached solid-phase drug particles and the space may need to be reduced and the adhesive force between particles and adhesive layer needs to be increased.

The particle-attached microneedles are prepared by attaching drug particles on the adhesive layered microneedle and the dissolution rate can be controlled by the size of attached particle. On the other hand, for conventional microneedles including coated microneedles and dissolving microneedles, additional additives such as a stabilizer, a thickener, and an excipient are added to prepare a liquid formulation for coating of molding solution. However, since the addition of additives for liquid formulation is not required for particle-attached microneedles. Thus, the preparation of particle-attached microneedles can be prepard more simply, and a target drug can be delivered into the skin without preparation of liquid formulation. Also the hydrophobic drug can be delivered into the skin without the dissolution in solvent.

According to the present disclosure, the method of manufacturing the particle-attached microneedles include a step of initial coating a microneedle with an adhesive solution to form an adhesive layer; and a step of attaching particles in petri-dish on the adhesive layered microneedle to secondarily attach solid-phase drug particles.

Before generating an adhesive layer on the uncoated microneedles, uncoated microneedles are made of a biodegradable polymer or non-biodegradable/biocompatible polymer(non-biodegradable but biocompatible polymer) and then an adhesive solution is prepared. Particle-attached microneedles are placed on the patch. The patch may have a circular shape, and an array of particle-attached microneedles may be placed on the patch. The microneedle may be inserted through the skin of a person, and after administration of particle-attached microneedles to the skin, the solid drug particles on the adhesive layer of particle-attached microneedle are dissolved and delivered into the skin.

A biodegradable polymer, which is a raw material of the uncoated microneedle, may be one or more selected from poly(lactic acid), poly(L-lactic acid), poly(glycolic acid) and poly(lactic-co-glycolic acid). Non-biodegradable/biocompatible polymer(non-biodegradable but biocompatible polymer) which is a raw material of the uncoated microneedle, may be one or more selected from a cyclic olefin copolymer, polycarbonate, nylon, polyethylene, and polypropylene In addition, the microneedle has a length of 50 $\mu$m to 1,000 $\mu$m, preferably a length of 400 $\mu$m to 1,000 $\mu$m. The length of the microneedle represents an appropriate length for the needle to reach the dermis layer through the stratum corneum of the skin.

Next, the method of forming (first coating) an adhesive layer on the uncoated microneedles may use the following three types.

First, a complex-type curable material and a hardener (the complex-type curable material is a curable resin),
Second, a single-type curable material (the single-type curable material is a liquid curable resin),
Third, an adhesive material with solvent (the adhesive material is a polymer).

For first and second case of curable adhesive material, the curing process for adhesive layer may be completed before drug particles are attached to an adhesive layer, or the curing process for adhesive layer may be completed after attaching the drug particles to an adhesive layer.

For third case of adhesive material with solvent, solvent is removed by drying and then particles are attached on the adhesive layer after removing solvent.

First, when the complex-type curable material and the hardener are used,
the complex-type curable material and the hardener may be a combination of polydimethylsiloxane (PDMS) and a silicone elastomer curing agent (hardener), a combination of a medical epoxy bond and a hardener, or the like.

Second, when the single-type curable material is used,
the single-type curable material does not have a separate a hardener, so after coating the single-type curable material on the microneedle, drug particles are attached thereto, and curing is controlled by temperature (heat) or UV. Examples of a single-type material whose curing is controlled by temperature include polyethyleneglycol (PEG), polyethylene oxide (PEO), beeswax, triglyceride, silicone, epoxy, protein bioadhesive and the like, and examples of a single-type material whose curing is controlled by UV include a UV-curable medical-grade epoxy resin, and the like.

When using the curable material as in the first and second methods, an additional solvent is not required, so that both hydrophilic and hydropobic drug particles can be attached on the adhesive layer.

Third, when a mixture of an adhesive material and a solvent is used, the adhesive material may be sugar 2-20%, glucose 25-50%, starch 1-4%, gelatin 1-4%, carboxymethylcellulose (CMC-Na) 1-4%, gum arabic 2-5%, or the like when the solvent is water, and since the solvent is water, it is suitable for attaching a hydrophobic drug. When the solvent is an organic solvent such as ethanol, the adhesive material may be a cellulose derivative (hydroxypropyl cellulose (HPC), hydroxypropylmethylcellulose (HPMC)) 1-4%, methyl cellulose (MC), ethyl cellulose 0.5-2%), polyvinylpyrrolidone (PVP) 2-5%, cyanoacrylate, a fibrin glue, a protein glue, a polyethylene glycol (PEG) hydrogel sealant, silicone, epoxy, protein bioadhesive, a mussel adhesive protein, or the like. Similarly, adhesion is exhibited within the above % ranges, and since the solvent is an organic solvent, it is suitable for attaching a hydrophilic drug.

When the complex-type curable material and the hardener are used together or the single-type curable material is used, a microneedle is dip-coated, brush-spread, or spray-coated with the complex-type curable material and the hardener or the single-type curable material, thereby forming an adhesive layer.

Since the adhesive solution before curing has sticky viscosity, the the dipping of uncoated microneedles in the curing solution for few seconds is recommended. After forming the adhesive layer, curing solution is cured in an oven at 70°C for 1 hour or at room temperature for one day to obtain an adhesive layer on an array bof microneedles. Alternatively, the adhesive layer may be solidified by irradiating ultraviolet (UV) light after using a UV curable resin as the single-type curable material.

To prepare solid-phase drug particles for secondary attachment of particles on adhesive layer, a solid-phase drug is dissolved in distilled water or buffer and then lyophilized. The lyophilized drug is pulverized with a mortar and passed through a micro sieve to prepare drug particles having a uniform shape and size. The drug particles passing through the micro sieve have a diameter of 20 $\mu$m or less.

Preferably, the drug particles have a diameter of 10 nm to 50 $\mu$m. More preferably, the drug particles may have a diameter of 1 $\mu$m to 20 $\mu$m, but the present disclosure is not limited thereto. The efficiency of delivery of particles through the microneedle can be affected by the size of the particles. If the particle size is too large, the amount of delivered particles may be decreased because the drug is pushed out due to increased friction between the particles and the skin during insertion into the skin. Conversely, if particles are too small, the amount of delivered particles may be decreased because the total volume of particles attached to the adhesive layer decreases.

An active material of the drug particles may be any material that can be medicamentally/medically used in the human body, such as a solidifying compound, an antibody, a drug substance such as a protein, and a vaccine. Specifically, the active material may be any one selected from the group consisting of interferon, erythropoietin (EPO), follicle-stimulating hormone (FSH), parathyroid hormone (PTH), granulocyte-macrophage colony-stimulating factor (G-CSF), granulocyte-macrophage colony-stimulating factor (FM-CSF), human chorionic gonadotropin, progesterone, calcitonin, glucagon, GNRH antagonist, insulin, human growth hormone (GHD), testosterone, lidocaine, diclofenac, oxybutynin, ketoprofen, alendronate, enapril maleate, phenylpropanolamine, cromolyn, isotretinoin, oxytocin, paroxetine, flurbiprofen, sertraline, venlafaxine, leuprolide, risperidone, galantamine, enoxaparin, etanercept, fentanyl, filgrastim, heparin, somatropin, and sumatriptan, or may be any one selected from the group consisting of a vaccine containing any one of Hepatitis A, B, and C, HIV, influenza, diphtheria, tetanus, pertussis, Lyme disease, rabies, pneumococcus, yellow fever, cholera, vaccinia, tuberculosis, rubella, measles, mumps, rotavirus, botulism, and herpes virus, and a botox toxin, and their combination.

After the step of secondarily attaching the solid-phase drug particles, a step of controlling void (space) between drug particles may be further included (reducing voids). In the present disclosure, controlling the voids between drug particles attached to microneedles means reducing the distance between drug particles.

The method of controlling the voids between the drug particles after attaching the drug particles on adhesive layer may be any one selected from among a method of treating the microneedle with air blow; a method of dip-coating the microneedle onto a puff; and a method of pressing the microneedle in a polydimethylsiloxane (PDMS) mold.

Specifically, the method of treating a microneedle with an air blow is a method of injecting air into a particle-attached microneedle. After this process, unattached drug particles to the adhesive layer are removed by air blow, and additional force for increase of adhesion between particles and adhesive layer was generated by air blow.

In the method of dip-coating a microneedle onto a puff, a puff is a porous structure with voids thereinside like a sponge, and when a particle-attached microneedle that has not been treated with air blow is pressed with a puff, large voids between the drug particles are compacted by the puff so that the distance between the particles can be reduced.

In the method of pressing a microneedle in a polydimethylsiloxane (PDMS) mold, the PDMS mold refers to an engraved PDMS mold used to manufacture a microneedle 10. Since the size and shape of the microneedle 10 and the PDMS mold perfectly fit each other, the drug particles are compacted and the voids between the particles are reduced when the particle-attached microneedle that is not treated with air blow is properly combined with the engraved part of the PDMS mold.

Through the step of controlling the voids between the drug particles, the solid-phase drug particles may be coated more thinly and evenly on the surface of the microneedle. Through this manufacturing method, a particle-attached microneedle including the adhesive layer formed by first coating the microneedle with the adhesive solution and the layer formed by secondarily coating the solid-phase drug particles on the adhesive layer is manufactured. Similarly, the particle-attached microneedle is in a state in which voids between the secondarily coated solid-phase drug particles are controlled. By controlling the voids, a large amount of drug particles may be attached to the same area, and since the drug particles are attached closer to the surface of the microneedle, the risk of the particles being pushed out when applied to the skin may be reduced.

Since the particle-attached microneedle according to the present disclosure is formed by coating drug particles on the microneedle, a desired dissolution rate may be determined by controlling the particle size. On the other hand, in an existing microneedle coating method, additional additives such as a stabilizer, a thickener, and an excipient are added to solid-phase drug particles to prepare a liquid formulation for coating, and it is necessary to change the formulation to make a solution. However, since this formulation change process is not required if the particles themselves are coated on the microneedle according to the present disclosure, the microneedle coating may be performed more simply, and a desired drug may be delivered at it is without changing a formulation. In addition, there is no need for a liquefaction process of dissolving a solid-phase drug in a buffer to make a liquid formulation for coating, so particle coating may be performed regardless of the hydrophilicity or lipophilicity of the drug.

Hereinafter, the present disclosure will be described in more detail through examples. These examples are for describing the present disclosure in more detail, and the scope of the present disclosure is not limited by these examples. In the following preparation examples, comparative examples, and examples, solid-phase drug particles were replaced with a solid-phase powder.

**[Preparation Example 1] Solid-phase powder particles**

A solid-phase powder containing silica and corn starch was prepared.

The powder was dissolved in tertiary distilled water at a concentration of 100 mg/mL, and then lyophilized.

The lyophilized powder was pulverized with a mortar, and then passed through a micro sieve having a mesh size of 10 $\mu$m, thereby manufacturing powder particles having a uniform shape and size.

FIG. 2 is an image of the powder particles according to the preparation example observed using a scanning transfer microscope (SEM) of 900 magnification. Referring to this, it is confirmed that the average diameter of the powder particles is $7.5 \pm 2.52$ $\mu$m.

**[Preparation Example 2] PDMS 10**

PDMS and a silicone elastomer curing agent were mixed at a weight ratio of 10:1, and then placed in a vacuum for 10 minutes to remove air bubbles, then poured into a flat mold and placed in a vacuum for 5 minutes to remove air bubbles.

The mold was first cured in an oven at 70°C for 40 minutes and then cooled to manufacture a film.

The first-cured film was separated from the mold and secondly cured in an oven at 190°C for 10 minutes.

**[Preparation Example 3] PDMS 40**

An experiment was carried out in the same manner as in Preparation Example 2 except that PDMS and a silicone elastomer curing agent were mixed in a weight ratio of 40:1.

[Table 1]

| Preparation Example 1 | Solid-phase powder containing silica and corn starch |
|---|---|
| Preparation Example 2 | PDMS 10 (weight ratio of PDMS to silicone elastomer curing agent is 10:1) |
| Preparation Example 3 | PDMS 40 (weight ratio of PDMS to silicone elastomer curing agent is 40:1) |

**[Comparative example] Manufacture of PLA microneedle first coated with PDMS**

Three poly lactic acid (PLA) pellets were placed on a PDMS mold modeled using a mold and melted in an oven at 190°C for 60 minutes. Next, after repeating the process of pressing PLA with a certain force while cooling PLA three times, PLA was removed from the mold to produce a PLA microneedle having 145 pyramid-shaped needle tips and a patch diameter of 1.2 cm.

After mixing PDMS and a silicone elastomer curing agent in a weight ratio of 40:1 to prepare an adhesive solution, the adhesive solution was applied to the PLA microneedle using a brush (brush spreading).

The PLA microneedle coated with the adhesive solution was cured in an oven at 70°C for 60 minutes, and then cooled, thereby manufacturing a microneedle first coated with the adhesive layer.

Referring to the enlarged part of the needle in FIG. 3B, it can be confirmed that the needle surface is coated with an adhesive layer. This was taken as a stereomicroscopic image and illustrated in FIG. 4A.

**[Example 1] Powder coating microneedle - ends of needle tips**

After placing the PDMS film manufactured in Preparation Example 2 on a coating well of a depth of 300 $\mu$m, the powder prepared in Preparation Example 1 was evenly dispersed and filled in the coating well. When a powder was secondarily coated on the microneedle manufactured in Comparative Example using a micro-scale coating equipment (one dipping coating for one second at a speed of 20 mm/s), only the ends of the tips of the microneedle were powder-coated.

At a distance of 10 cm from the coated microneedle, air compressor wind was sprayed on all four sides of the microneedle for 3 seconds each. A microneedle whose tip ends were only powder-coated was manufactured by blowing off a non-adherent powder.

**[Example 2] Powder coating microneedle - entire needle**

After filling a petri dish with the powder prepared in Preparation Example 1, the microneedle prepared in the comparative example was completely immersed therein, thereby being secondarily coated.

At a distance of 10 cm from the coated microneedle, air compressor wind was sprayed on all four sides of the microneedle for 3 seconds each. A non-adherent powder was blown off so that the entire microneedle including the patch surface on which the needle was located was secondarily coated.

The comparative example and Examples 1 and 2 are summarized in Table 2 below.

[Table 2]

| Classification | **Comparative example** | **Example 1** | **Example 2** |
|---|---|---|---|
| **First PDMS coating** | O | O | O |
| **Secondarily powder coating** | X | Entirety of needle | Needle tip ends |

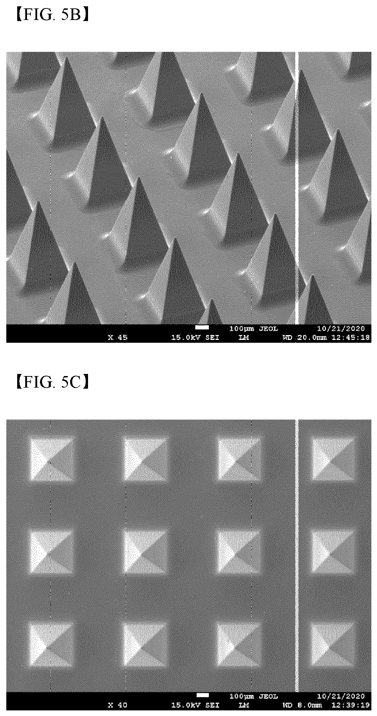

FIGS. 5A to 5C illustrate SEM images of a PLA microneedle before first coating with an adhesive layer in the comparative example. Here, FIG. 5A illustrates a front perspective view observed at 43 magnification, FIG. 5B illustrates a side perspective view observed at 45 magnification, and FIG. 5C illustrates a plan perspective view observed at 40 magnification. Each tip of the microneedle has a pyramid shape, a length of 700 $\mu$m, and a bottom width of 500 $\mu$m, and the interval between the needle tips is 280 $\mu$m.

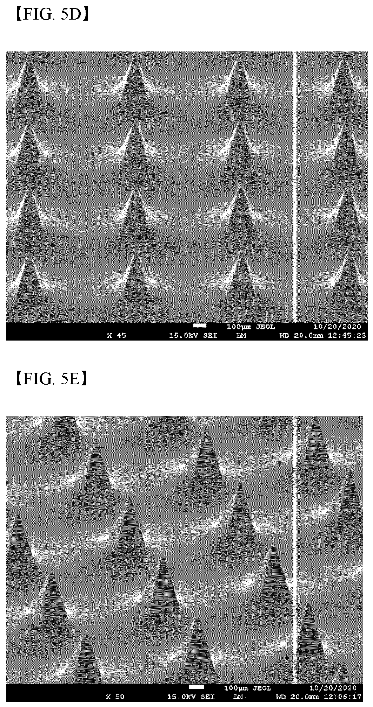

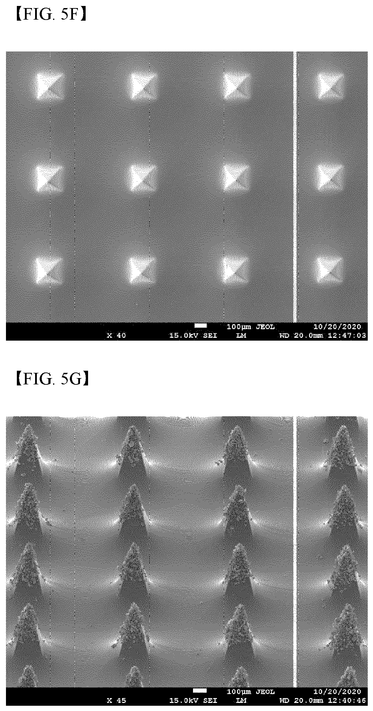

FIGS. 5D to 5F illustrate SEM images of the microneedle after first coating with the adhesive layer in the comparative example. Here, FIG. 5D illustrates a front perspective view observed at 45 magnification, FIG. 5E illustrates a side perspective view observed at 50 magnification, and FIG. 5F illustrates a plan perspective view observed at 40 magnification. Referring to the drawings, it can be confirmed that the coating of the PDMS adhesive layer of a ratio of 40:1 was uniformly made.

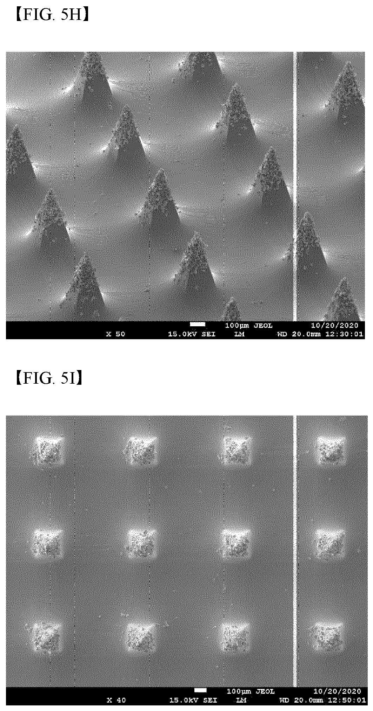

FIGS. 5G to 5I illustrate SEM images of a microneedle whose needle tip ends were only secondarily coated with particles, after first coating with the adhesive layer in the comparative example. Here, FIG. 5G illustrates a front perspective view observed at 45 magnification, FIG. 5H illustrates a side perspective view observed at 50 magnification, and FIG. 5I illustrates a plan perspective view observed at 40 magnification. Referring to the drawings, it can be confirmed that particles were uniformly coated on only ends of the needle tips on which the PDMS adhesive layer had been coated.

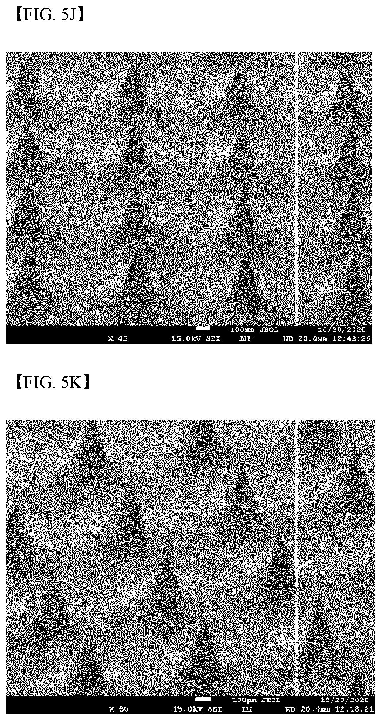

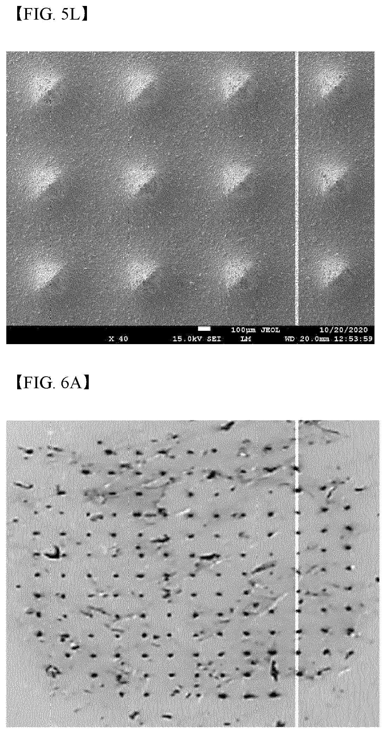

FIGS. 5J to 5L illustrate SEM images of a microneedle, the entirety of which was secondarily coated with particles after first coating with the adhesive layer in the comparative example. Here, FIG. 5J illustrates a front perspective view observed at 45 magnification, FIG. 5K illustrates a side perspective view observed at 50 magnification, and FIG. 5L illustrates a plan perspective view observed at 40 magnification. Referring to the drawings, it can be confirmed that particles were uniformly coated on the entirety of the needle on which the PDMS adhesive layer had been coated.

**[Experimental Example 1]**

A frozen pig skin (full skin) was thawed at room temperature for 1 hour, and then was flatly fixed on a wooden board using a nail.

The microneedle (Example 1) whose tip ends were only powder-coated, and the microneedle (Example 2), the entirety of which was powder-coated, were respectively placed on the pig skin, and a vertical force of 40N to 50N was applied thereto.

Hereinafter, results obtained using the experimental example are described.

**<Transmittance>**

A microneedle was removed with tweezers, and the skin where the microneedle had been placed was stained by applying 10 μL of 0.4% Trypan blue solution thereto with a micropipette.

After 10 minutes, the Trypan blue solution was wiped off with a cotton swab dipped in a phosphate-buffered saline (PBS) buffer.

The stained skin surface was photographed under a light microscope. This process was repeated 5 times to produce Samples Nos. 1 to 5.

When the total number (145) of needles was 100%, the results expressed in % by counting the number of permeated holes are shown in Tables 3 and 4 below.

[Table 3] First coating with PDMS adhesive layer + particles being attached only to needle tip ends

| Sample No. | 1 | 2 | 3 | 4 | 5 | Average (%) | Standard deviation |
|---|---|---|---|---|---|---|---|
| Transmittance (%) | 100 | 100 | 100 | 100 | 100 | 100 | 0.00 |

[Table 4] First coating with PDMS adhesive layer + particles being attached to entirety of needle

| Sample No. | 1 | 2 | 3 | 4 | 5 | Average (%) | Standard deviation |
|---|---|---|---|---|---|---|---|
| Transmittance (%) | 100 | 100 | 100 | 100 | 100 | 100 | 0.00 |

In Table 3, the microneedle of Example 1 was used. When the microneedle of Example 1 was applied to pig skin, a permeability was 100% in all samples. FIG. 6A illustrates the skin through which Sample No. 1 penetrated.

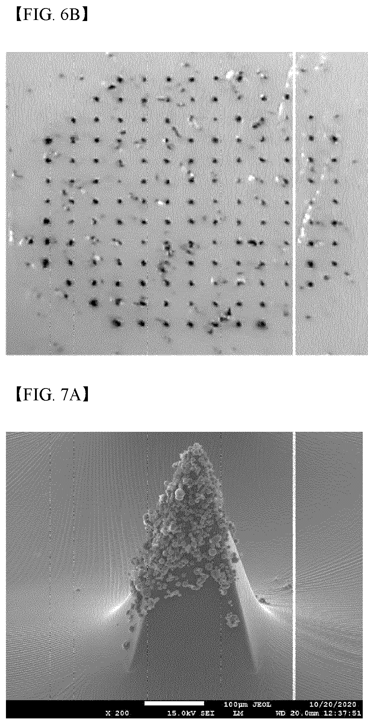

In Table 4, the microneedle of Example 2 was used. Also when the microneedle of Example 2 was applied to pig skin, a permeability was 100% in all samples. FIG. 6B illustrates the skin through which Sample No. 1 penetrated.

When comparing the two particle attachment methods, a skin permeability of 100% was observed in both the methods. Therefore, it can be seen that the particle-attached microneedles prepared in Examples 1 and 2 had sufficient strength and sharpness to penetrate the stratum corneum of the skin and deliver a drug to the epidermal layer or the dermal layer as a target.

**< Degree of peeling of microneedle-adhesive layer >**

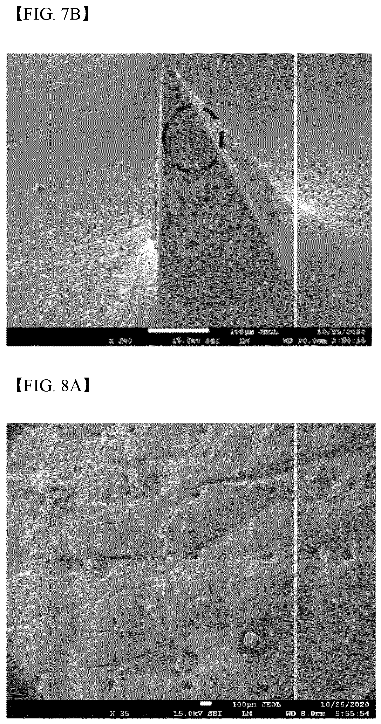

FIGS. 7A and 7B are scanning electron microscope (SEM) images, taken at 200 times magnification, illustrating the peeling degree of the first-coated adhesive layer of Example 1 (only needle tip ends were powder-coated).

FIG. 7A illustartes an image of a microneedle before piercing the microneedle into pig skin. It can be seen that the powder is attached to the PDMS adhesive layer.

FIG. 7B illustartes an image of the microneedle after piercing the microneedle into pig skin once. As can be seen from the part indicated in the drawing, it can be confirmed that the particle adhesion of the needle is maintained and the adhesive layer is not peeled off.

**< Extruded powder form >**

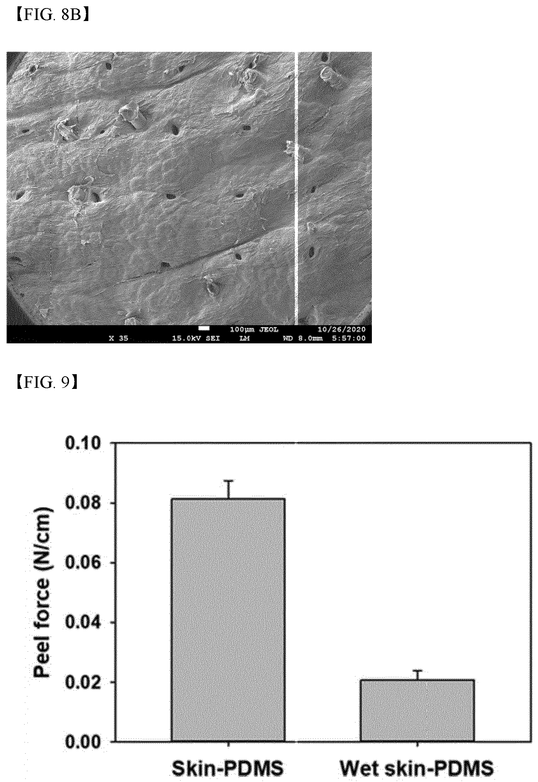

FIG. 8A is a control and illustrates an image of the surface of pig skin observed after stabbing a first-coated microneedle that was not coated with particles. FIG. 8B is an experimental group and illustrates pig skin stabbed with Example 1 (after coating the microneedle with the PDMS adhesive layer, particles were attached to only the tip ends of the microneedle). When the image of the experimental group is compared with the image of the control, it can be seen that the almost no particles were smeared around the region where the microneedle was stabbed, and very few particles were peeled off to the skin surface after administering the particle-attached microneedle.

**[Experimental Example 2] Measurement of peel force of PDMS 40**

Using Preparation Example 3, peel force on pig skin was compared.

To measure the peeling force of PDMS on dry pig skin, a PDMS 40 film was prepared and pig skin (ex vivo) having a width of 2.5 cm was placed on the film. Next, the pig skin was pressed onto the PDMS 40 film with a pressure of 3 kg.

To measure the peeling force of PDMS on wet pig skin, glucose particles were applied to a PDMS 40 film, and pig skin (ex vivo) having a width of 2.5 cm was placed on the particle film. Next, the pig skin was pressed with a pressure of 3 kg.

The peel force was measured at a 90° angle by a direct pull off method using a stress-strain tester (ZwickiLine, ZwickRoell, Germany). For both the samples, the tip of the pig skin was connected to a metal holder and the holder was connected to a sensor with a string. The pig skin was deformed at a constant rate of 10 mm/min.

When the microneedle is removed from the skin after administration, the adhesion force between PDMS 40 and poly(lactic acid) should be greater than the adhesion force between PDMS 40 and the wet skin and the repulsive force caused by friction. When the adhesion force between PDMS 40 and the wet skin is greater than the adhesion force between PDMS 40 and the poly(lactic acid) microneedle surface, the adhesive layer, PDMS 40, peels off during administration or removal and remains on the skin, so it may have harmful effects on the human body.

When the microneedle is administered to the pig skin, PDMS 40 is exposed to interstitial fluid present in the skin, and the adhesion of PDMS 40 is reduced. Therefore, after inserting the microneedle into the skin, the adhesive force between the PDMS and the attached solid-phase particles decreases, resulting in rapid separation of the solid-phase particles from the surface of the PDMS adhesive layer. During the time of application of the microneedle to the skin, the adhesion between the solid-phase particles and the PDMS should be high enough to overcome the friction between the solid-phase particles and the skin.

FIG. 9 is a graph illustrating the peel force between PDMS 40 and pig skin. "Skin-PDMS" indicates the peel force between PDMS 40 and dry pig skin, and "Wet skin-PDMS" indicates the peel force between PDMS 40 and wet pig skin. Referring to FIG. 9, since the adhesion of PDMS 40 to wet skin is rapidly decreased, it can be expected that the frictional force with PDMS to wet skin will also be lowered. Therefore, it is possible to safely remove the microneedle from the skin without high friction between the PDMS 40 and the skin.

**[Experimental Example 3] Horizontal work of PDMS 40**

The horizontal work between glucose particles and pig skin was compared with the horizontal work between a PDMS 40 film and pig skin.

Pig skin (2.5 cm × 2.5 cm, W × L) was placed on a PDMS 40 film (5 cm × 5 cm, W × L, thickness=0.5 cm), and a block weighing 100 g was placed on the pig skin. One end of the string was attached to the pig skin and the other end of the string was attached to the force sensor. Next, the pig skin was pulled in a direction parallel to the PDMS 40 film at a speed of 10 mm/min.

Glucose particles with an average diameter of 10 $\mu$m were attached to a PDMS 40 film (5 cm×5 cm, W×L, thickness=0.5 cm). Pig skin (2.5 cm × 2.5 cm, W × L) was placed on the particle layer. A 100 g weight block was placed on the pig skin. The shear force between the glucose particles and the skin was measured by pulling the pig skin tangentially to the film at a speed of 10 mm/min.

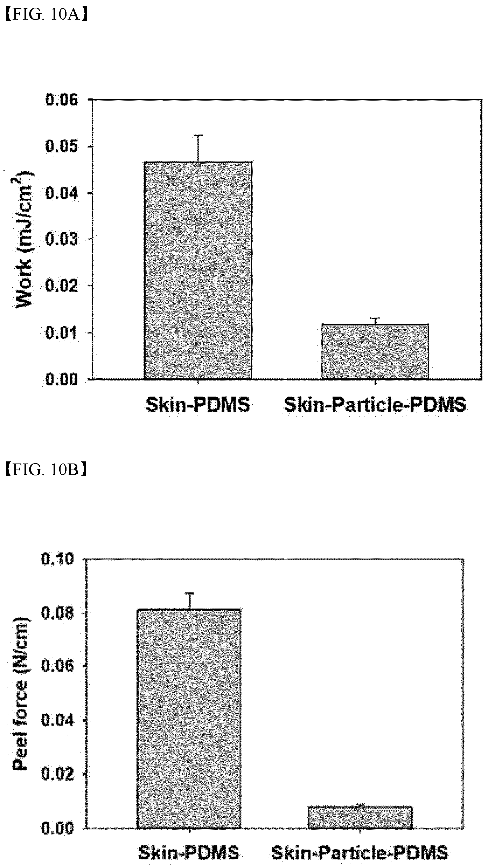

FIG. 10A is a graph illustrating the horizontal work between PDMS 40 and pig skin.

Referring to FIG. 10A, when a load of 100 g was applied to the specimen, the horizontal work (exprssed as "Skin-PDMS") between PDMS 40 and skin was 0.048 mJ/cm$^2$, and when glucose particles were attached to PDMS 40, the horizontal work (exprssed as "Skin-Particle-PDMS") bewteen the surface of the glucose particles and the skin was 0.0125 mJ/cm$^2$. That is, when glucose particles were present at the interface between PDMS 40 and the skin, the horizontal work was significantly reduced. A decrease in horizontal work means a decrease in frictional resistance by the skin when microneedles penetrate into the skin.

**[Experimental Example 4] Peel force of PDMS 40**

FIG. 10B is a graph illustrating the peel force between PDMS 40, glucose particles, and pig skin.

Referring to FIG. 10B, the peel force of glucose particles from pig skin (expressed as "Skin-Particle-PDMS") is about 25% of the peel force of PDMS 40 from pig skin (expressed as "Skin-PDMS"). From the fact that the peel force was reduced by the glucose particles, it can be seen that the resistance by the skin is reduced when the microneedle is administered, so that the glucose particles will be administered in a well-attached state to the adhesive surface. In addition, the glucose particles reduce the resistance caused by the skin, thereby reducing the frictional force delivered to the PDMS 40 adhesive layer. The adhesion force of PDMS 40 to the poly(lactic acid) microneedles is not affected by skin friction.

**[Experimental Example 5] Adhesion force of ovalbumin particles to PDMS40**

Ovalbumin particles with an average diameter of 10 $\mu$m were attached to a plate with a diameter of 0.5 cm containing a PDMS 40 adhesive layer. Next, the plate was placed in a centrifuge tube. After centrifuging the tube at 27,000 g (Centrifuge, 1730R, LABOGENE, South Korea) for 10 min, the plate was weighed using a high-precision balance (Satorius SE2-F, Göttingen, Germany), and the amount of ovalbumin particles separated from the surface of PDMS 40 during centrifugation was calculated.

When the PDMS 40 plate to which ovalbumin particles were attached was rotated with an acceleration of 27,000 g, the pressure applied to the 10 $\mu$m-thick plate (1 cm × 1 cm) was 0.2 N/cm$^2$, and 96% of the initial ovalbumin particles remained on the surface of PDMS 40. Most of the particles were not separated from the surface of PDMS 40 because the vertical adhesion force between the ovalbumin particles and PDMS 40 was more than 0.2 N/cm$^2$. If the solid-phase particles do not adhere well to the surface of the PDMS 40 adhesive layer, the particles are pushed back by the skin and the amount absorbed into the skin decreases.

**[Experimental Example 6] Ovalbumin particle delivery efficiency to skin dependent upon particle size**

A particle transfer efficiency was observed using a microneedle to which ovalbumin particles were attached. The microneedle to which ovalbumin particles have been attached was inserted into pig skin ex vivo and left at 37 °C for 5 minutes, and then removed from the skin. The initial amount of ovalbumin was measured using an ovalbumin ELISA kit (LSBio, Seattle, WA). A cotton swab was moistened with distilled water and the injection site was wiped therewith. Next, the removed microneedle was immersed in 1 ml of distilled water, and the cotton swab was also immersed in 1 ml of distilled water for 10 minutes. The amount of ovalbumin remaining was measured using an ovalbumin ELISA kit. The amount of ovalbumin delivered to the skin was calculated by subtracting the amount of residual ovalbumin from the amount of ovalbumin initially loaded into the microneedle. A microneedle to which particles having each of three diameters (less than 1 μm, 1 to 20 μm, and greater than 20 μm) had been attached was inserted into the ex vivo pig skin and the amount of ovalbumin particles delivered to the skin was measured. The drug delivery efficiency is defined as the amount delivered to the skin, divided by the total amount of drug coated, multiplied by 100.

As mentioned above, the frictional force between the solid-phase particles and the skin surface is an important factor that separates the solid-phase particles from the surface of the PDMS 40 adhesive layer during microneedle insertion.

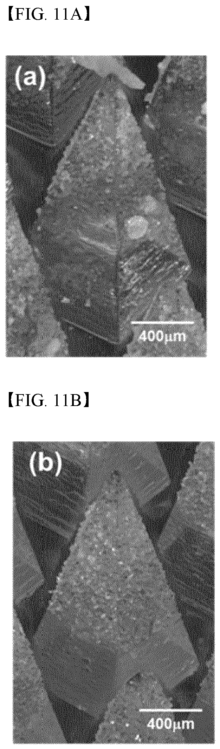

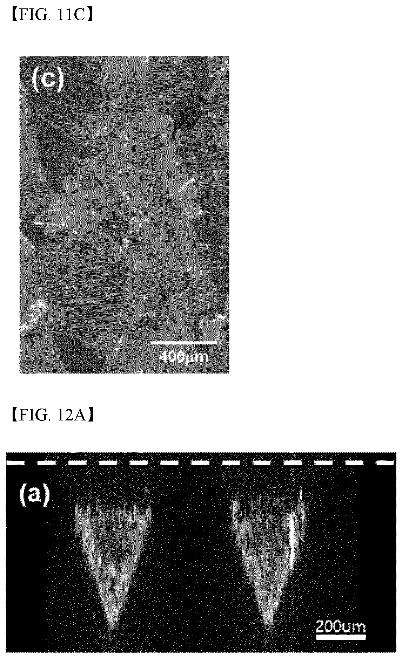

FIGS. 11A to 11C illustrate images in which ovalbumin particles of different sizes are attached to a PDMS 40 adhesive layer. Referring to FIG. 11A, in the case of nanoparticles with a diameter of less than 1 μm, aggregates were easily formed in the particles, and aggregates, instead of individual particles, were attached to the surface of PDMS 40. Referring to FIG. 11C, in the case of particles having a diameter of more than 20 μm, the surface of the microneedle was very rough due to the large particles. Referring to FIG. 11B, particles with a diameter of 1 to 20 μm formed a uniform coating layer on the surface of the PDMS adhesive layer of the microneedle.

Table 5 below shows an initial ovalbumin particle amount (The initial amount of particle) and particle transfer efficiency (Delivery efficiency), which are dependent upon an ovalbumin particle diameter (Size of particles in diameter), of a particle-attached microneedle in which the height of a PDMS 40 adhesive layer is 700 μm.

As shown in Table 5, it can be seen that the delivery efficiency varies depending upon the size of the ovalbumin particles. When the particle size was more than 20 μm, the geometric friction between the particles and the skin increased, which decreased the transfer efficiency. That is, as the particle size increased, the separation of particles from the surface of the adhesive layer increased. On the other hand, when the particle size decreased, the friction force decreased. However, if the particle size in the fixed adhesive layer region decreases , the amount of particles delivered to the skin may decrease.

[Table 5]

| Size of particles in diameter (μm) | The initial amount of particle (μg/array) | Delivery efficiency (%) |
|---|---|---|
| Below 1 | 302.0 ± 15 | 93 (± 0.86) |
| 1-20 | 412 ± 19.5 | 94 (± 1.48) |
| Above 20 | 660 ± 20 | 86 (± 2.11) |

$$* \text{Delivery efficiency (\%)} = (\llbracket \text{The initial amount of particle} \rrbracket\_before - \llbracket \text{The amount of particle} \rrbracket\_(after)) / \llbracket \text{The amount of particle} \rrbracket\_(before) \times 100$$

Delivered amount = Initial amount of particles before - The amount of remaining particles after The amount of remaining particles after = The amount remaining on microneedle surface after +the amount remaining on skin surface after

**[Experimental Example 7] In vitro delivery characteristics of solid-phase particles**

**[Experimental Example 7-1] Confirmation of delivery characteristics of ovalbumin particles administered to skin**

The particle transfer properties of the microneedle to which the solid particles were attached were observed. A microneedle to which fluorescent ovalbumin particles were attached was inserted into pig skin for 5 minutes. Using a confocal microscope (ECLIPSE TE2000-E, Nikon, Osaka, Japan), the fluorescence image and fluorescence change of the microneedle administration site were observed with a Z-stack over time. In addition, the threshold of fluorescence intensity in pig skin was determined and the boundary of the fluorescence region within the threshold was defined. The pattern of decrease in fluorescence intensity over time was quantified using ImageJ software (NIH ImageJ; NIH, Bethesda, MD).

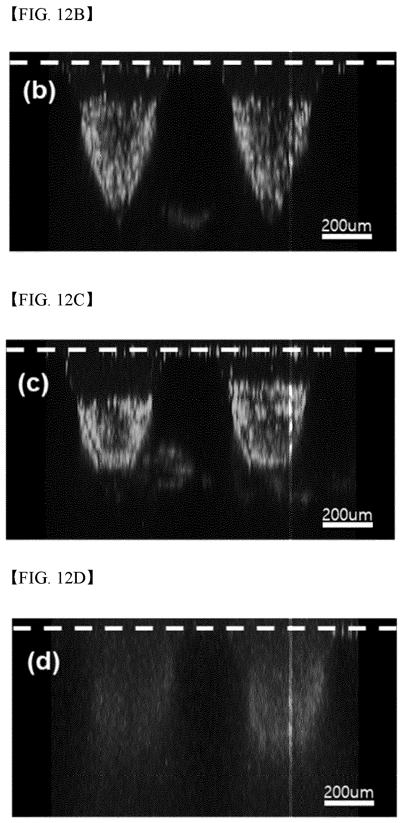

FIGS. 12A to 12D illustrate images of observing the Z-stack fluorescence pattern of pig skin over time using a confocal microscope. FIG. 12A is an image immediately after administration, FIG. 12B is an image when 1 minute has elapsed after administration, FIG. 12C is an image when 3 minutes have elapsed after administration, and FIG. 12D is an image when 5 minutes have elapsed after administration.

Referring to FIGS. 12A to 12D, a conventional coated microneedle and dissolving microneedles show a smooth fluorescent surface, whereas the particle-attached microneedle has agglomerated clusters of fluorescent particles upon initial administration. In addition, unlike the exponential decrease in fluorescence intensity that occurs in the conventional coated microneedle and the dissolving microneedle, the fluorescence intensity of the particle-attached microneedle shows a semi-log pattern that decreases with time.

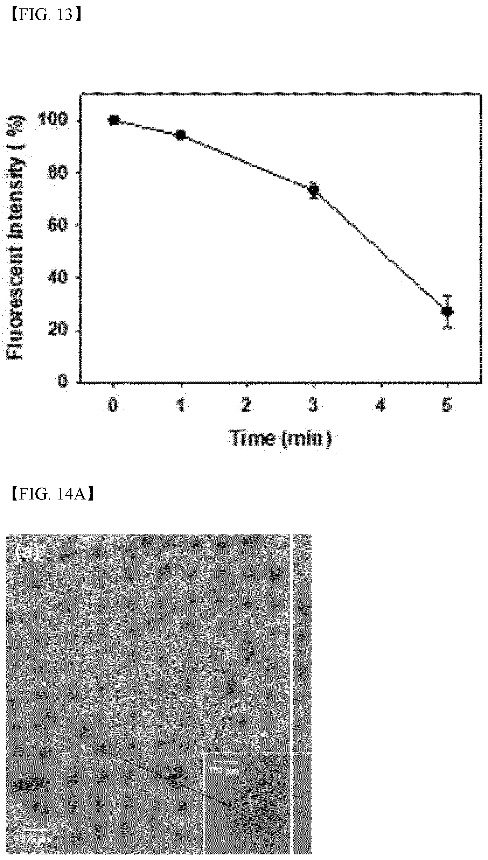

FIG. 13 is a graph illustrating a change in fluorescence intensity on the surface of a tip of a microneedle while the microneedle to which fluorescent ovalbumin particles are attached stays on the skin. Referring to FIG. 13, the fluorescence intensity of the particle-attached microneedle decreased by 70% within 5 minutes. In the case of dissolving microneedles and conventional coated microneedle using a water-soluble drug formulation, it takes about 40 minutes for the fluorescence intensity to decrease by 70%. Therefore, the microneedle to which the fluorescent ovalbumin particles are attached has the advantage of being able to rapidly deliver the ovalbumin particles to the skin.

**[Experimental Example 7-2] Confirmation of diffusion of glucose and ovalbumin particles dissolved in skin**

A microneedle with green glucose particles and a microneedle with fluorescent ovalbumin particles were inserted into the pig skin ex vivo for 5 minutes and removed. The skin surface was wiped with a wet cotton swab to remove skin residues, and after 10 minutes, the pig skin surface was observed using an optical microscope and a fluorescence microscope, respectively.

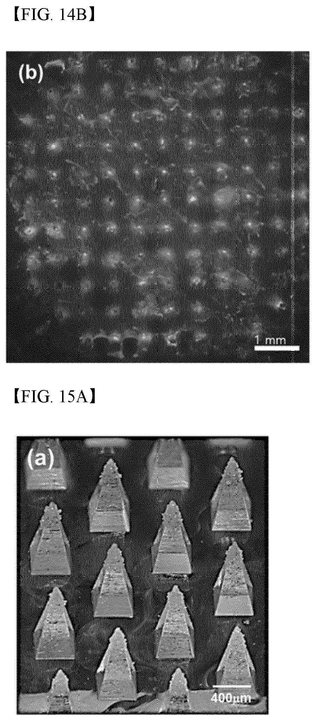

FIG. 14A illustrates an optical microscope image of a microneedle to which green glucose particles are attached. FIG. 14B illustrates a fluorescence microscope image of a microneedle to which fluorescent ovalbumin particles are attached.

Referring to FIGS. 14A and 14b, it can be confirmed that the green glucose particles and the fluorescent ovalbumin particles were successfully delivered to the skin 5 minutes after the microneedle to which green glucose particles had been attached and the microneedle to which fluorescent ovalbumin particles had been attached was inserted.

In FIG. 14A , the lower right image is an enlarged image. The inner circle indicates the initial administration position of the particle, and the outer circle indicates the diffusion of green glucose particles after the microneedle is removed.

**[Experimental Example 8] Comparison of particle-attached microneedle vs intramuscular injection**

**[Experimental Example 8-1] Measurement of in vivo delivery efficiency of ovalbumin particles**

To attach 20 $\mu$g of ovalbumin particles, the number of microneedle tips per microneedle patch and the coating height of the PDMS adhesive layer were adjusted to 57 and 230 $\mu$m, respectively. The ovalbumin particles were filled in a well with a depth of 0.3 mm and coated secondarily. Non-adherent ovalbumin particles were removed using an air compressor (6 L/min). The microneedle patch to which ovalbumin particles had been attached was dissolved in 4 ml of distilled water, and then the concentration of ovalbumin was measured using the Bradford protein analysis method.

After inserting the microneedle with ovalbumin particles into female BALB/c mice (4-6 weeks old; Orient Bio, Seongnam, South Korea) for 5 minutes, the surface of the microneedles was optically observed using a microscope. The administration site was covered with adhesive tape. The recovered microneedle and tape were placed in distilled water. The amount of ovalbumin was measured using the ovalbumin ELISA kit. The amount of delivered ovalbumin particles was indirectly measured by subtracting the total amount of ovalbumin remaining on the microneedle surface and mouse skin surface from the amount of initially attached ovalbumin.

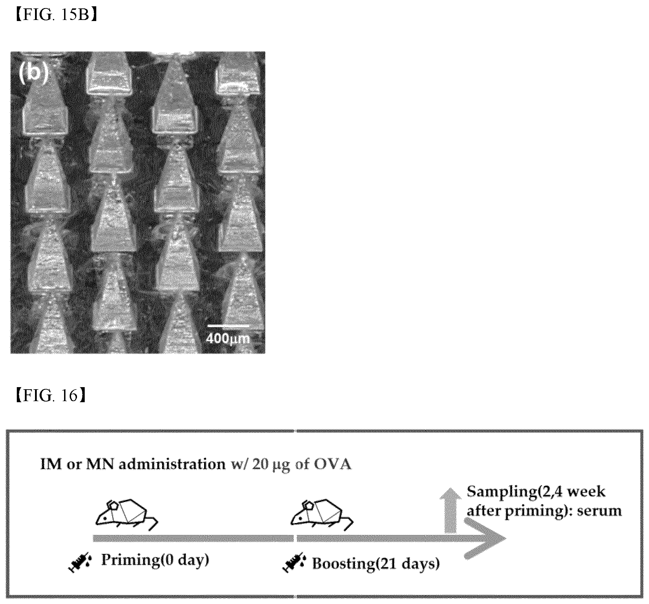

FIG. 15A illustrates an optical microscope image of a microneedle to which ovalbumin particles are attached, and FIG. 15B illustrates an optical microscope image of a microneedle, to which ovalbumin particles are attached, recovered after administering the microneedle to mouse skin in vivo for 5 minutes.

Referring to FIG. 15B, no ovalbumin particles were found on the surface of the recovered microneedle. As a result of administering the microneedle, to which 20 $\mu$g of ovalbumin was attached, to a mouse for 5 minutes, the particle transfer efficiencies of the priming experiment and the boosting experiment (see FIG.16) were 90.9% and 92.3%, respectively. That is, the delivery efficiency of ovalbumin particles according to the in vivo mouse experiment was almost the same as that of the ovalbumin particles according to the in vitro experiment (see Table 5).

**[Experimental Example 8-2] Comparison of immune efficacy**

FIG. 16 illustrates a schematic diagram of an immunization schedule. Female BALB/c mice (4-6 weeks old; Orient Bio, Seongnam, South Korea) were inoculated using an ovalbumin-coated microneedle according to the schedule of FIG. 16. BALB/c mice were primed with ovalbumin (20 $\mu$g/mouse) dissolved in distilled water (DW) using intramuscular injection (indicated by "IM"). After 3 weeks (21 days), the mice were boosted with ovalbumin (20 $\mu$g/mouse) dissolved in distilled water. $\mu$g/mouse). In addition, the microneedle (represented as "Particle-attached microneedles") to which 20 $\mu$g ovalbumin particles had been attached was administered to mice on the same day as above. Serum was collected at 14 days (2 weeks) and 28 days (4 weeks) after priming as indicated for immune response. The administration time of the ovalbumin particle-attached microneedle for priming and boosting was 5 minutes.

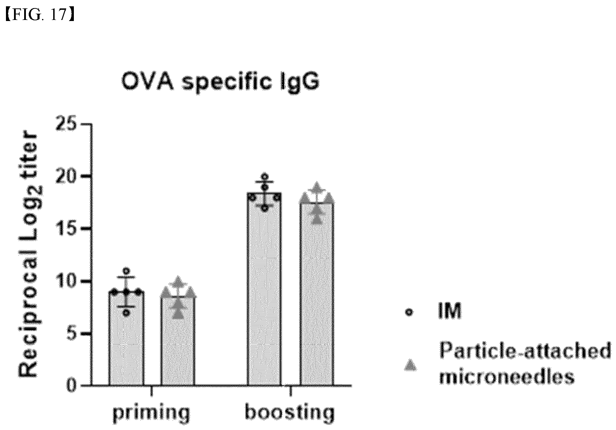

Ovalbumin-specific antibody titers were analyzed by ELISA. FIG. 17 illustrates a graph of antibody titer when ovalbumin is administered. Referring to FIG. 17, ovalbumin delivery using the ovalbumin particle-attached microneedle induced an antigen-specific immune response similar to that of intramuscular administration of ovalbumin dissolved in distilled water. These results indicate that the particle-attached microneedle is an efficient delivery method for vaccination.

The antibody titer deviation by the intramuscular injection (IM) was similar to the antibody titer deviation of the particle-attached microneedle. These results were obtained because the deviation between the amount of ovalbumin delivered by the 5-minute administration of the particle-attached microneedle and the amount of ovalbumin delivered by intramuscular injection was small.

The particle-attached microneedle is a novel microneedle system that uses a water-free process. The system includes secondarily coating (adhering) a solid-phase drug onto an adhesive layer that is first coated on the microneedle. Unlike existing microneedle systems, the particle-attached microneedle does not require a liquid formulation, and solid-phase drug particles are physically attached to the outside of the microneedle. The particle-attached microneedle allows multiple drugs to be delivered simultaneously by attaching multiple solid-phase drugs to a single microneedle patch.

For successful intradermal administration of a solid-phase drug, the adhesion force of the adhesive layer applied to particles should be greater than the friction force of the particles to the skin. In the present disclosure, an adhesion force sufficient to overcome the friction between the particles and the skin was generated by appropriately increasing the mixing ratio of a curable material and a hardener.

When the adhesion force between the solid-phase drug and the adhesive layer is reduced and the particles outside the microneedles are exposed to the fluid in the skin, the time required for a particle-attached microneedle to remain on the skin may be reduced. The adhesion amount of the particles is controlled by adjusting the area of the adhesive layer. Ovalbumin particles with an average diameter of 10 $\mu$m were delivered to pig skin for 5 minutes with a delivery efficiency of 93% in vitro and to mouse skin with a delivery efficiency of 90% or more in vivo. Finally, a mouse experiment using the microneedle to which ovalbumin particles were attached showed an ovalbumin antibody titer value similar to that produced by intramuscular administration.

The method of producing the microneedle first coated with an adhesive layer includes a simple manufacturing process of loading a solid-phase drug on the outside of the microneedle without changing the intrinsic properties of the drug. In addition, the particle-attached microneedle can provide a solution to overcome the limitations of multivalent vaccines and mixed drugs. The particle-attached microneedle can be applied to a wide range of applications for drug and vaccine administration.

In accordance with the present disclosure, solid drug particles can be used without modification.

In accordance with the present disclosure, when the solid drug is loaded onto the microneedle, the drug activity can be maintained because there is no need for additional additives, such as a liquid formulation, other than drug particles, and no drying process is required.

In accordance with the present disclosure, since the drug particles on the microneedle are delivered directly to the dermis, the additive does not affect the dissolution and dissolution patterns of the drug.

Although the present disclosure has been described through limited examples and figures, the present disclosure is not intended to be limited to the examples. Those skilled in the art will appreciate that various modifications, additions and substitutions are possible, without departing from the scope of the disclosure. The invention is defined by the appended claims.

[Description of Symbols]

10: microneedle
11: adhesive layer
20: coating well
21: film
22: solid-phase drug particles

## Claims

1. A method of manufacturing a particle-attached microneedle, the method comprising:

   first coating a microneedle with an adhesive solution to form an adhesive layer; and
   dipping the microneedle, on which the adhesive layer has been formed, in a coating well filled with solid-phase drug particles to secondarily attach the solid-phase drug particles on the adhesive layer.

2. The method according to claim 1, further comprising disposing a film on the coating well to disperse the solid-phase drug particles before filling the solid-phase drug particles in the coating well.

3. The method according to claim 1, further comprising, after secondarily attaching the solid-phase drug particles, controlling voids between the secondarily attached solid-phase drug particles, wherein controlling voids means reducing the distance between drug particles.

4. The method according to claim 3, wherein the microneedle secondarily attached with the solid-phase drug particles is treated with air blow, which is a method of injecting air by which unattached drug particles to the adhesive layer are removed by air blow, and additional force for increase of adhesion between particles and adhesive layer is generated by air blow, to control voids between the solid-phase drug particles and increase adhesion between the particles and adhesive layer.

5. The method according to claim 3, wherein the microneedle secondarily attached with the solid-phase drug particles is pressed on a porous structure to control voids between the solid-phase drug particles.

6. The method according to claim 3, wherein the microneedle secondarily attached with the solid-phase drug particles is pressed in a polydimethylsiloxane (PDMS) mold to control voids between the solid-phase drug particles.

7. The method according to claim 1, wherein the adhesive solution comprises a complex-type curable material and a hardener,

   wherein the complex-type curable material is polydimethylsiloxane (PDMS) or a medical epoxy bond, and
   the hardener is a silicone elastomer curing agent or a hardener.

8. The method according to claim 7, wherein a weight ratio of the complex-type curable material and hardener comprised in the adhesive solution is 40:1 to 20:1.

9. The method according to claim 1, wherein the adhesive solution comprises a single-type curable material, and the single-type curable material is any one selected from the group consisting of polyethyleneglycol (PEG), polyethylene oxide (PEO), beeswax, triglyceride, silicone, epoxy, protein bioadhesive and a UV-curable medical-grade epoxy resin.

10. The method according to claim 1, wherein the adhesive solution comprises an adhesive material and a solvent,

   wherein the adhesive material is any one selected from the group consisting of sugar 2-20%, glucose 25-50%, starch 1-4%, gelatin 1-4%, carboxymethylcellulose (CMC-Na) 1-4%, gum arabic 2-5%, cellulose derivative (hydroxypropyl cellulose (HPC), hydroxypropylmethylcellulose (HPMC)) 1-4%, methyl cellulose (MC), ethyl cellulose 0.5-2%), polyvinylpyrrolidone (PVP) 2-5%, cyanoacrylate, a fibrin glue, a protein glue, a polyethylene glycol (PEG) hydrogel sealant, silicone, epoxy, protein bioadhesive and a mussel adhesive protein, and the solvent is water or an organic solvent.

11. The method according to claim 1, wherein a raw material of the microneedle is one or more selected from biodegradable polymers and non-biodegradable/biocompatible polymers,

wherein biodegradable polymers consist of poly(lactic acid), poly(L-lactic acid), poly(glycolic acid), poly(lactic-co-glycolic acid) and polycaprolactone,

wherein non-biodegradable/biocompatible polymers consist of cyclic olefin copolymer, polycarbonate, nylon, polyethylene and polypropylene.

12. The method according to claim 1, wherein the microneedle has a length of 50 $\mu$m to 1,000 $\mu$m.

13. The method according to claim 1, wherein an active material of the solid-phase drug particles comprise any one or more selected from the group consisting of interferon, erythropoietin (EPO), follicle-stimulating hormone (FSH), parathyroid hormone (PTH), granulocyte-macrophage colony-stimulating factor (G-CSF), granulocyte-macrophage colony-stimulating factor (FM-CSF), human chorionic gonadotropin, progesterone, calcitonin, glucagon, GNRH antagonist, insulin, human growth hormone (GHD), testosterone, lidocaine, diclofenac, oxybutynin, ketoprofen, alendronate, enapril maleate, phenylpropanolamine, cromolyn, isotretinoin, oxytocin, paroxetine, flurbiprofen, sertraline, venlafaxine, leuprolide, risperidone, galantamine, enoxaparin, etanercept, fentanyl, filgrastim, heparin, somatropin, and sumatriptan, or comprises any one selected from the group consisting of a vaccine containing any one of Hepatitis A, B, and C, HIV, influenza, diphtheria, tetanus, pertussis, Lyme disease, rabies, pneumococcus, yellow fever, cholera, vaccinia, tuberculosis, rubella, measles, mumps, rotavirus, botulism, and herpes virus, and a botox toxin.

14. A method of manufacturing a particle-attached microneedle, the method comprising:

first coating a microneedle with an adhesive solution to form an adhesive layer; and
dispersing solid-phase drug particles on the adhesive layer using a micro-sieve to secondarily attach the solid-phase drug particles on the adhesive layer.

15. The method according to claim 14, further comprising, after secondarily attaching the solid-phase drug particles, controlling voids between the secondarily attached solid-phase drug particles,
wherein controlling voids means reducing the distance between drug particles.

16. A method of manufacturing a particle-attached microneedle, the method comprising:

first coating a microneedle with an adhesive solution to form an adhesive layer; and
dipping the microneedle, on which the adhesive layer has been formed, in a petri dish filled with solid-phase drug particles to secondarily attach the solid-phase drug particles on the adhesive layer.

17. The method according to claim 16, further comprising, after secondarily attaching the solid-phase drug particles, controlling voids between the secondarily attached solid-phase drug particles,
wherein controlling voids means reducing the distance between drug particles.

**Patentansprüche**

1. Verfahren zur Herstellung einer partikelbefestigten Mikronadel, wobei das Verfahren umfasst:

zunächst Beschichten einer Mikronadel mit einer Klebstofflösung, um eine Klebstoffschicht zu bilden; und
Eintauchen der Mikronadel, auf der die Klebstoffschicht gebildet wurde, in einen mit Festphasenarzneimittelpartikeln gefüllten Beschichtungsbehälter, um die Festphasenarzneimittelpartikel sekundär an der Klebstoffschicht zu befestigen.

2. Verfahren nach Anspruch 1, ferner umfassend Aufbringen eines Films auf den Beschichtungsbehälter, um die Festphasenarzneimittelpartikel vor dem Einfüllen der Festphasenarzneimittelpartikel in den Beschichtungsbehälter zu dispergieren.

3. Verfahren nach Anspruch 1, ferner umfassend, nach dem sekundären Befestigen der Festphasenarzneimittelpartikel, Regulieren von Lücken zwischen den sekundär befestigten Festphasenarzneimittelpartikeln, wobei das Regulieren von Lücken bedeutet, den Abstand zwischen Arzneimittelpartikeln zu verringern.

4. Verfahren nach Anspruch 3, wobei die sekundär mit den Festphasenarzneimittelpartikeln angebrachte Mikronadel mit einem Luftstoß behandelt wird, welcher ein Verfahren zum Injizieren von Luft ist, durch das unbefestigte Arzneimittelpartikel an der Klebstoffschicht mittels Luftstoß entfernt werden, und durch den Luftstoß eine zusätzliche Kraft zur Erhöhung von Adhäsion zwischen Partikeln und Klebstoffschicht erzeugt wird, um Lücken zwischen den Festphasenarzneimittelpartikeln zu regulieren und die Adhäsion zwischen den Partikeln und der Klebstoffschicht zu erhöhen.

5. Verfahren nach Anspruch 3, wobei die mit den Festphasenarzneimittelpartikeln sekundär befestigte Mikronadel, auf eine poröse Struktur gedrückt wird, um Lücken zwischen den Feststoffarzneimittelpartikeln zu regulieren.

6. Verfahren nach Anspruch 3, wobei die mit den Festphasenarzneimittelpartikeln sekundär befestigte Mikronadel in eine Polydimethylsiloxan-(PDMS-)Form gepresst wird, um Lücken zwischen den Festphasenarzneimittelpartikeln zu regulieren.

7. Verfahren nach Anspruch 1, wobei die Klebstofflösung ein aushärtbares Material komplexen Typs und einen Härter umfasst,

   wobei das aushärtbare Material komplexen Typs Polydimethylsiloxan (PDMS) oder eine medizinische Epoxidverbindung ist, und
   der Härter ein Silikon-Elastomer-Härtungsmittel oder ein Härter ist.

8. Verfahren nach Anspruch 7, wobei ein Gewichtsverhältnis des von der Klebstofflösung umfassten aushärtbaren Materials komplexen Typs und des Härters 40:1 bis 20:1 ist.

9. Verfahren nach Anspruch 1, wobei die Klebstofflösung ein aushärtbares Material eines einzigen Typs umfasst, und das aushärtbare Material eines einzigen Typs irgendeines ist, ausgewählt aus der Gruppe bestehend aus Polyethylenglykol (PEG), Polyethylenoxid (PEO), Bienenwachs, Triglycerid, Silikon, Epoxid, ProteinBioadhäsiv und einem UV-härtbaren Epoxidharz medizinischer Qualität.

10. Verfahren nach Anspruch 1, wobei die Klebstofflösung ein Klebstoffmaterial und ein Lösungsmittel umfasst, wobei das Klebstoffmaterial irgendeines ist, ausgewählt aus der Gruppe bestehend aus Zucker 2-20 %, Glukose 25-50 %, Stärke 1-4 %, Gelatine 1-4 %, Carboxymethylcellulose (CMC-Na) 1-4 %, Gummi arabicum 2-5 %, Zellulosederivat (Hydroxypropylcellulose (HPC), Hydroxypropylmethylcellulose (HPMC)) 1-4 %, Methylcellulose (MC), Ethylcellulose 0,5-2 %), Polyvinylpyrrolidon (PVP) 2-5 %, Cyanoacrylat, einem Fibrinkleber, einem Proteinkleber, einem Polyethylenglykol-(PEG-)Hydrogeldichtmittel, Silikon, Epoxid, Proteinbioadhäsiv und einem Muscheladhäsivprotein, und das Lösungsmittel Wasser oder ein organisches Lösungsmittel ist.

11. Verfahren nach Anspruch 1, wobei ein Rohstoff der Mikronadel einer oder mehrere ist, ausgewählt aus biologisch abbaubaren Polymeren und nicht biologisch abbaubaren/biokompatiblen Polymeren,

   wobei die biologisch abbaubaren Polymere bestehen aus Poly(milchsäure), Poly(L-Milchsäure), Poly(glykolsäure), Poly(milch-co-glykolsäure) und Polycaprolacton,
   wobei die nicht biologisch abbaubaren/biokompatiblen Polymere bestehen aus zyklischem Olefin-Copolymer, Polycarbonat, Nylon, Polyethylen und Polypropylen.

12. Verfahren nach Anspruch 1, wobei die Mikronadel eine Länge von 50 $\mu$m bis 1.000 $\mu$m hat.

13. Verfahren nach Anspruch 1, wobei ein Wirkstoff der Festphasenarzneimittelpartikel einen oder mehrere umfasst, ausgewählt aus der Gruppe, bestehend aus Interferon, Erythropoietin (EPO), Follikelstimulierendes Hormon (FSH), Nebenschilddrüsenhormon (PTH), Granulozyten-Makrophagen-Kolonie-stimulierendem Faktor (G-CSF), Granulozyten-Makrophagen-Kolonie-stimulierendem Faktor (FM-CSF), humanem Choriongonadotropin, Progesteron, Calcitonin, Glukagon, GNRH-Antagonist, Insulin, menschlichem Wachstumshormon (GHD), Testosteron, Lidocain, Diclofenac, Oxybutynin, Ketoprofen, Alendronat, Enaprilmaleat, Phenylpropanolamin, Cromolyn, Isotretinoin, Oxytocin, Paroxetin, Flurbiprofen, Sertralin, Venlafaxin, Leuprolid, Risperidon, Galantamin, Enoxaparin, Etanercept, Fentanyl, Filgrastim, Heparin, Somatropin und Sumatriptan, oder irgendeines umfasst, ausgewählt aus der Gruppe bestehend aus einem Impfstoff, enthaltend irgendeines von Hepatitis A, B und C, HIV, Influenza, Diphtherie, Tetanus, Keuchhusten, Lyme-Krankheit, Tollwut, Pneumokokken, Gelbfieber, Cholera, Vaccinia, Tuberkulose, Röteln, Masern, Mumps, Rotavirus, Botulismus, Herpesvirus und einem Botox-Toxin.

14. Verfahren zur Herstellung einer partikelbefestigten Mikronadel, wobei das Verfahren umfasst:

zuerst Beschichten einer Mikronadel mit einer Klebstofflösung, um eine Klebstoffschicht zu bilden; und Dispergieren von Festphasenarzneimittelpartikeln auf der Klebstoffschicht unter Verwendung eines Mikrosiebs zur sekundären Befestigung der Festphasenarzneimittelpartikel auf der Klebstoffschicht.

15. Verfahren nach Anspruch 14, ferner umfassend, nach dem sekundären Befestigen der Festphasenarzneimittelpartikel, Regulieren von Lücken zwischen den sekundär befestigten Festphasenarzneimittelpartikeln. wobei Regulieren von Lücken bedeutet, den Abstand zwischen Arzneimittelpartikeln zu verringern.

16. Verfahren zur Herstellung einer partikelbefestigten Mikronadel, wobei das Verfahren umfasst:

zuerst Beschichten einer Mikronadel mit einer Klebstofflösung, um eine Klebstoffschicht zu bilden; und Eintauchen der Mikronadel, auf der die Klebstoffschicht gebildet wurde, in eine mit Festphasenarzneimittelpartikeln gefüllte Petrischale, um die Festphasenarzneimittelpartikel sekundär an der Klebstoffschicht zu befestigen.

17. Verfahren nach Anspruch 16, ferner umfassend, nach dem sekundären Befestigen der Festphasenarzneimittelpartikel, das Regulieren von Lücken zwischen den sekundär befestigten Festphasenarzneimittelpartikeln, wobei Regulieren von Lücken bedeutet, den Abstand zwischen Arzneimittelpartikeln zu verringern.


**Revendications**

1. Procédé de fabrication d'une micro-aiguille à particules fixées, le procédé comprenant :

en premier lieu le revêtement d'une micro-aiguille avec une solution adhésive pour former une couche adhésive ; et
l'immersion de la micro-aiguille, sur laquelle la couche adhésive a été formée, dans un puits de revêtement rempli de particules de médicament en phase solide pour fixer de manière secondaire les particules de médicament en phase solide sur la couche adhésive.

2. Procédé selon la revendication 1, comprenant en outre la mise en place d'un film sur le puits de revêtement pour disperser les particules de médicament en phase solide avant l'introduction des particules de médicament en phase solide dans le puits de revêtement.

3. Procédé selon la revendication 1, comprenant en outre, après avoir fixé de manière secondaire les particules de médicament en phase solide, le contrôle des vides entre les particules de médicament en phase solide fixées de manière secondaire,
dans lequel le contrôle des vides signifie la réduction de la distance entre les particules de médicament.

4. Procédé selon la revendication 3, dans lequel la micro-aiguille à laquelle sont fixées de manière secondaire les particules de médicament en phase solide est traitée par soufflage d'air, qui est un procédé d'injection d'air grâce auquel les particules de médicament non fixées à la couche adhésive sont éliminées par soufflage d'air, et une force supplémentaire pour augmenter l'adhérence entre les particules et la couche adhésive est générée par soufflage d'air, pour contrôler les vides entre les particules de médicament en phase solide et augmenter l'adhérence entre les particules et la couche adhésive.

5. Procédé selon la revendication 3, dans lequel la micro-aiguille à laquelle sont fixées de manière secondaire les particules de médicament en phase solide est pressée sur une structure poreuse pour contrôler les vides entre les particules de médicament en phase solide.

6. Procédé selon la revendication 3, dans lequel la micro-aiguille à laquelle sont fixées de manière secondaire les particules de médicament en phase solide est pressée dans un moule de polydiméthylsiloxane (PDMS) pour contrôler les vides entre les particules de médicament en phase solide.

7. Procédé selon la revendication 1, dans lequel la solution adhésive comprend un matériau polymérisable de type complexe et un durcisseur,

dans lequel le matériau polymérisable de type complexe est du polydiméthylsiloxane (PDMS) ou une colle époxy à usage médical, et
le durcisseur est un agent de polymérisation pour élastomère de silicone ou un durcisseur.

8. Procédé selon la revendication 7, dans lequel un rapport de poids du matériau polymérisable de type complexe et d'un durcisseur compris dans la solution adhésive est de 40:1 à 20:1.

9. Procédé selon la revendication 1, dans lequel la solution adhésive comprend un matériau polymérisable de type simple, et
le matériau polymérisable de type simple est l'un quelconque sélectionné dans le groupe consistant en polyéthylèneglycol (PEG), oxyde de polyéthylène (PEO), cire d'abeille, triglycéride, silicone, époxy, bioadhésif protéique et une résine époxy de qualité médicale polymérisable aux UV.

10. Procédé selon la revendication 1, dans lequel la solution adhésive comprend un matériau adhésif et un solvant,

dans lequel le matériau adhésif est l'un quelconque sélectionné dans le groupe consistant en du sucre 2 à 20 %, du glucose 25 à 50 %, de l'amidon 1 à 4 %, de la gélatine 1 à 4 %, du carboxymethylcellulose (CMC-Na) 1 à 4 %, de la gomme arabique 2 à 5 %, un dérivé de cellulose (hydroxypropyl cellulose (HPC), hydroxypropylméthylcellulose (HPMC) 1 à 4 %, méthylcellulose (MC), éthylcellulose 0,5 à 2 %), de la polyvinylpyrrolidone (PVP) 2 à 5 %, du cyanoacrylate, une colle à la fibrine, une colle protéique, un agent de scellement hydrogel à base de polyéthylène glycol (PEG), du silicone, de l'époxy, un bioadhésif protéique et une protéine moulière adhésive, et
le solvant est de l'eau ou un solvant organique.

11. Procédé selon la revendication 1, dans lequel une matière première de la micro-aiguille est un ou plusieurs polymères sélectionnés parmi des polymères biodégradables et des polymères non-biodégradables/biocompatibles,

dans lequel les polymères biodégradables consistent en acide polylactique, acide poly(L-lactique), acide polyglycolique, acide polylactique-co-glycolique et polycaprolactone,
dans lequel les polymères non-biodégradables/biocompatibles consistent en copolymère de cyclooléfine, polycarbonate, nylon, polyéthylène et polypropylène.

12. Procédé selon la revendication 1, dans lequel la micro-aiguille a une longueur de 50 μm à 1000 μm.

13. Procédé selon la revendication 1, dans lequel un matériau actif des particules de médicament en phase solide comprend un ou plusieurs éléments quelconques sélectionnés dans le groupe consistant en interféron, érythropoïetine (EPO), hormone folliculo-stimulante (FSH), hormone parathyroïdienne (PTH), facteur de stimulation des colonies de granulocytes et de macrophages (G-CSF), facteur de stimulation des colonies de granulocytes et de macrophages (FM-CSF), gonadotropine chorionique humaine, progestérone, calcitonine, glucagon, antagoniste de la GnRH, insuline, hormone de croissance humaine (GHD), testostérone, lidocaïne, diclofénac, oxybutynine, kétoprofène, alendronate, maléate d'énapril, phénylpropanolamine, acide cromoglycique, isotrétinoïne, oxytocine, paroxétine, flurbiprofène, sertraline, venlafaxine, leuprolide, rispéridone, galantamine, énoxaparine, étanercept, fentanyl, filgrastim, héparine, somatropine et sumatriptan ou comprend l'un quelconque sélectionné dans le groupe consistant en un vaccin contenant l'un quelconque parmi le virus de l'hépatite A, B et C, du VIH, de la grippe, de la diphtérie, du tétanos, de la coqueluche, de la maladie de lyme, de la rage, du pneumocoque, de la fièvre jaune, du choléra, de la vaccine, de la tuberculose, de la rubéole, de la rougeole, des oreillons, du rotavirus, du botulisme et de l'herpès, et une toxine botulique.

14. Procédé de fabrication d'une micro-aiguille à particules fixées, le procédé comprenant :

en premier lieu, le revêtement d'une micro-aiguille avec une solution adhésive pour former une couche adhésive ; et

la dispersion de particules de médicament en phase solide sur la couche adhésive à l'aide d'un micro-tamis pour fixer de manière secondaire les particules de médicament en phase solide sur la couche adhésive.

15. Procédé selon la revendication 14, comprenant en outre, après avoir fixé de manière secondaire les particules de médicament en phase solide, le contrôle des vides entre les particules de médicament en phase solide fixées de manière secondaire,
dans lequel le contrôle des vides signifie la réduction de la distance entre les particules de médicament.

16. Procédé de fabrication d'une micro-aiguille à particules fixées, le procédé comprenant :

en premier lieu, le revêtement d'une micro-aiguille avec une solution adhésive pour former une couche adhésive ; et
l'immersion de la micro-aiguille, sur laquelle la couche adhésive a été formée, dans une boîte de Petri remplie de particules de médicament en phase solide pour fixer de manière secondaire les particules de médicament en phase solide sur la couche adhésive.

17. Procédé selon la revendication 16, comprenant en outre, après avoir fixé de manière secondaire les particules de médicament en phase solide, le contrôle des vides entre les particules de médicament en phase solide fixées de manière secondaire,
dans lequel le contrôle des vides signifie la réduction de la distance entre les particules de médicament.

【FIG. 1A】

① Manufacture needle

② Form adhesive layer (first coating)

③ Attach particles (second coating)

【FIG. 1B】

First coat microneedle with adhesive solution to form adhesive layer

- Brush spreading
- Spraying

Form adhesive film on entire needle

(i) Fill a coating well of a desired height with solid-phase drug particles, and then dip-coat the microneedle, on which an adhesive layer has been formed, to secondarily coat with the drug particles

(ii) Scatter drug particles on microneedle through a micro-sieve, or secondarily coat the entire microneedle with drug particles using a Petri dish

Particles attached only to needle tip

Particles attached to entire needle

- Use coating well

Form adhesive film only on needle tip

Secondarily coat drug particles on the microneedle, on which the adhesive layer has been formed to a desired height, using a coating well, a micro-sieve, or a Petri dish

Particles attached only to needle tip

【FIG. 2】

【FIG. 3A】

【FIG. 3B】

【FIG. 3C】

【FIG. 4A】

【FIG. 4B】

【FIG. 4C】

【FIG. 5A】

【FIG. 5B】

【FIG. 5C】

【FIG. 5D】

【FIG. 5E】

【FIG. 5F】

【FIG. 5G】

【FIG. 5H】

【FIG. 5I】

【FIG. 5J】

【FIG. 5K】

【FIG. 5L】

【FIG. 6A】

【FIG. 6B】

【FIG. 7A】

【FIG. 7B】

【FIG. 8A】

【FIG. 8B】

【FIG. 9】

【FIG. 10A】

【FIG. 10B】

【FIG. 11A】

【FIG. 11B】

【FIG. 11C】

【FIG. 12A】

【FIG. 12B】

【FIG. 12C】

【FIG. 12D】

【FIG. 13】

【FIG. 14A】

【FIG. 14B】

【FIG. 15A】

【FIG. 15B】

【FIG. 16】

IM or MN administration w/ 20 µg of OVA

Sampling(2,4 week after priming): serum

Priming(0 day)    Boosting(21 days)

【FIG. 17】

OVA specific IgG

**REFERENCES CITED IN THE DESCRIPTION**

**Patent documents cited in the description**

- KR 101785766
- US 20140005606 A1
- US 20050197308 A1